# EUROPEAN PATENT APPLICATION

(11) **EP 3 812 448 A1**
(43) Date of publication of application: **28.04.2021**
(21) Application number: 18926371.8
(22) Date of filing: 12.07.2018
(51) Int. Cl.: C12M 1/34, G01N 21/17

(54) **INFORMATION PROCESSING DEVICE, INFORMATION PROCESSING METHOD, PROGRAM, AND INFORMATION PROCESSING SYSTEM**

(71) Applicant: Sony Corporation, 108-0075 Tokyo (JP)
(72) Inventor: SHINODA, Masataka, Tokyo 140-0002 (JP)
(74) Representative: D Young & Co LLP
(86) International application number: PCT/JP2018/026390
(87) International publication number: WO 2020/012616

(57) **Abstract**

It is possible to deal with cells having ability to divide more appropriately while further reducing cost required for analyzing captured images of the cells having ability to divide.

Provided is an information processing apparatus including an analyzing unit configured to analyze a plurality of images obtained by capturing images of an observation target including a cell having ability to divide in chronological order, to calculate a feature amount representing a state of the cell, and a control unit configured to dynamically control an implementation condition of the analysis on a basis of the calculated feature amount.

## Description

### Field

The present disclosure relates to an information processing apparatus, an information processing method, a program, and an information processing system.

### Background

A technology of evaluating states of specimens using images of the specimens obtained by measuring the specimens such as cells is known in related art. However, there is a case where states of some specimens are inappropriate for measurement. The following Patent Literature 1 discloses a technology of specifying specimens which are in a state inappropriate for measurement by analyzing images of the specimens and avoiding measurement of the specimens in a state which is inappropriate for measurement in the future.

### Citation List

### Patent Literature

Patent Literature 1: JP 2017-62497 A

### Summary

### Technical Problem

Analysis of captured images of cells using a computer involves various types of cost including a running cost of calculators of the computer (such as a graphics processing unit (GPU) and a central processing unit (CPU)), which is generated in accordance with an analysis time during which the computer analyzes the images.

According to the technology disclosed in the above-described Patent Literature 1, measurement of specimens specified as specimens which are in a state inappropriate for measurement is avoided in the future, and thus a measurement time is shortened. However, according to the technology disclosed in Patent Literature 1, as a result of no measurement being performed in the future for the specimens which are not measurement targets, there occur specimens for which images are not obtained. There can be some specimens whose states are temporarily unfavorable, but may improve thereafter. Therefore, with the technology disclosed in Patent Literature 1, there is a possibility that specimens which should be essentially evaluated are excluded from evaluation.

The present disclosure therefore proposes a new and improved information processing apparatus, information processing method, program, and information processing system which is capable of dealing with cells having ability to divide more appropriately while further reducing cost required for analyzing captured images of the cells having ability to divide.

### Solution to Problem

According to the present disclosure, an information processing apparatus is provided that includes: an analyzing unit configured to analyze a plurality of images obtained by capturing images of an observation target including a cell having ability to divide in chronological order, to calculate a feature amount representing a state of the cell; and a control unit configured to dynamically control an implementation condition of the analysis on a basis of the calculated feature amount.

Moreover, according to the present disclosure, an information processing method to be performed by a processor provided at a calculator is provided that includes: analyzing a plurality of images obtained by capturing images of an observation target including a cell having ability to divide in chronological order, to calculate a feature amount representing a state of the cell; and dynamically controlling an implementation condition of the analysis on a basis of the calculated feature amount.

Moreover, according to the present disclosure, a program is provided that causes a computer to execute: analyzing a plurality of images obtained by capturing images of an observation target including a cell having ability to divide in chronological order, to calculate a feature amount representing a state of the cell; and dynamically controlling an implementation condition of the analysis on a basis of the calculated feature amount.

Moreover, an information processing system is provided that includes: an imaging apparatus including at least an imaging unit configured to generate a plurality of images regarding an observation target including a cell having ability to divide by capturing images of the observation target in chronological order; and an information processing apparatus including an analyzing unit configured to analyze the plurality of captured images to calculate a feature amount representing a state of the cell, and a control unit configured to dynamically control an implementation condition of the analysis on a basis of the calculated feature amount.

According to the present disclosure, the control unit provided at the information processing apparatus can dynamically control the implementation condition of image analysis on the basis of the feature amount of the cell having ability to divide. Further, the analyzing unit provided at the information processing apparatus can implement analysis of the captured image of the cell having ability to divide on the basis of the analysis implementation condition controlled by the control unit.

Further, according to the present disclosure, the control unit can exclude a certain cell having ability to divide on which attention is focused from targets of image analysis. However, images of even a cell which has been excluded from the targets of image analysis are continuously captured. The analyzing unit can therefore return even a cell which has been excluded from the targets of image analysis once to the targets of image analysis in accordance with control by the control unit.

### Advantageous Effects of Invention

As described above, according to the present disclosure, it is possible to deal with cells having ability to divide more appropriately while further reducing cost required for analyzing captured images of the cells having ability to divide.

### Brief Description of Drawings

FIG. 1 schematically illustrates a configuration of an information processing system according to an embodiment of the present disclosure.
FIG. 2 illustrates an example of a culture medium in which a plurality of fertilized eggs is cultured.
FIG. 3 is a functional block diagram illustrating a functional configuration example of an information processing apparatus according to an embodiment of the present disclosure.
FIG. 4 is a flowchart illustrating an example of processing to be performed by the information processing apparatus according to an embodiment of the present disclosure.
FIG. 5 is a flowchart for explaining flow of recognition analysis processing.
FIG. 6 is a flowchart for explaining flow of motion analysis processing.
FIG. 7 is a flowchart for explaining flow of morphological analysis processing.
FIG. 8 is a flowchart for explaining flow of numerical analysis processing.
FIG. 9 illustrates an example of a screen of a display apparatus on which information indicating an implementation status of image analysis is displayed.
FIG. 10A is a graph illustrating an example of relationship between an area of a fertilized egg with good quality and culture time.
FIG. 10B is a graph illustrating an example of a result obtained by an information processing apparatus in a first specific example calculating an area of a fertilized egg with poor quality.
FIG. 11A is a graph illustrating an example of relationship between an amount of motion of the whole fertilized egg with good quality and culture time.
FIG. 11B is a graph illustrating an example of a result obtained by an information processing apparatus in a second specific example calculating an amount of motion of the whole fertilized egg with poor quality.
FIG. 12A is a graph illustrating an example of relationship between growth stages of a fertilized egg with good quality and culture time.
FIG. 12B is a graph illustrating an example of a result obtained by an information processing apparatus in a third specific example calculating growth stages of a fertilized egg with poor quality.
FIG. 13 is a graph for explaining an aspect where an information processing apparatus in a fourth specific example stops calculation of feature amounts of fertilized eggs in a stepwise manner.
FIG. 14 is a graph for explaining an aspect where an information processing apparatus in a fifth specific example stops analysis methods to be used in image analysis of fertilized eggs in a stepwise manner.
FIG. 15 is a graph for explaining an aspect where an information processing apparatus in a sixth specific example interrupts image analysis of fertilized eggs with poor quality at predetermined culture time intervals.
FIG. 16 is a graph for explaining an aspect where an information processing apparatus in a seventh specific example interrupts image analysis of fertilized eggs with poor quality so that a period during which image analysis is not implemented becomes longer as time elapses.
FIG. 17 is a graph for explaining reduction of cost required for image analysis by the information processing apparatus according to an embodiment of the present disclosure.
FIG. 18 is a block diagram illustrating a hardware configuration example of the information processing apparatus according to an embodiment of the present disclosure.

### Description of Embodiments

Favorable embodiments of the present disclosure will be described in detail with reference to the appended drawings. In this specification and the drawings, components having substantially the same functional configuration are denoted by the same reference numerals, and redundant description is omitted.

Note that the description will be given in the following order.
1. Outline of information processing system
2. Information processing apparatus
2.1. Configuration example
2.2. Processing example
2.3. Effects
3. Hardware configuration example
4. Conclusion

### <<1. Outline of information processing system>>

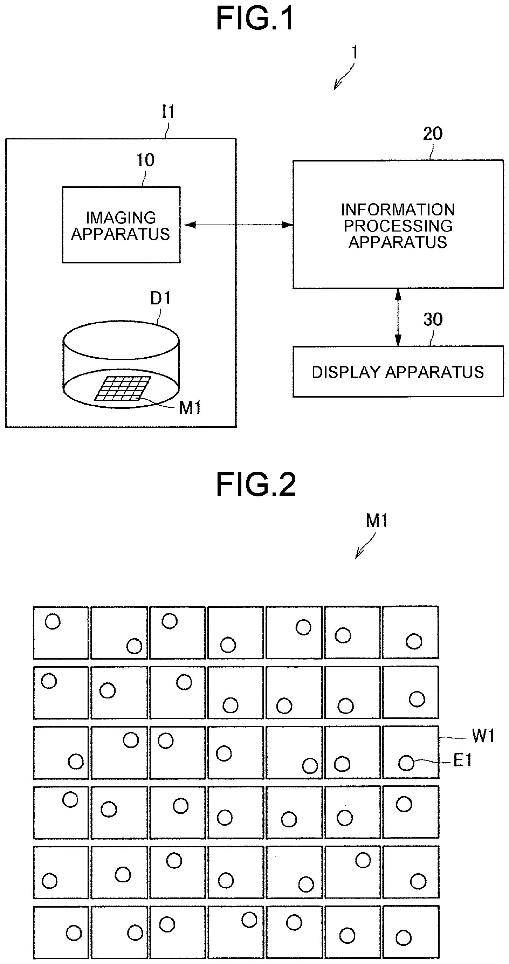

FIG. 1 schematically illustrates a configuration of an information processing system 1 according to an embodiment of the present disclosure. As illustrated in FIG. 1, the information processing system 1 includes an imaging apparatus 10, an information processing apparatus 20, and a display apparatus 30. The imaging apparatus 10, the information processing apparatus 20 and the display apparatus 30 are connected to each other via various kinds of wired or wireless networks. Further, the information processing apparatus 20 is connected to the Internet and can implement information processing and analysis processing on the cloud. Still further, the imaging apparatus 10, the information processing apparatus 20 and the display apparatus 30 may be directly connected to each other via predetermined connection terminals.

Note that, in the information processing system 1 according to an embodiment of the present disclosure, a cell which becomes an image capturing target is not particularly limited if the cell has ability to divide, and publicly known various kinds of cells can be set as the image capturing target. Examples of such a cell having ability to divide can include, for example, fertilized eggs of various kinds of organisms, a cancel cell, an ES cell, an iPS cell, and the like. Description will be provided below using a fertilized egg of an organism as a specific example of the cell having ability to divide.

Note that, in the present specification, the "fertilized egg" conceptually includes at least a single cell and an aggregate of a plurality of cells. Here, the single cell or the aggregate of a plurality of cells relate to cells observed in one or a plurality of stages of growth process of the fertilized egg, including an oocyte, an egg or an ovum, a fertile ovum or zygote, a blastocyst and an embryo.

### (Imaging apparatus)

The imaging apparatus 10 is an apparatus which generates images relating to a subject by capturing images of the subject in chronological order. The imaging apparatus 10 includes at least publicly known various kinds of imaging elements such as a charge coupled device (CCD) or a complementary metal oxide semiconductor (CMOS) sensor. The imaging apparatus 10 may include various kinds of members such as a lens for forming an image of the subject at the imaging element and a light source to irradiate the subject with illumination light in addition to these imaging elements.

As illustrated in FIG. 1, the imaging apparatus 10 according to an embodiment of the present disclosure is, for example, provided above a dish D1 having a culture medium M1 in which fertilized eggs which are observation targets are cultured, inside an incubator I1 for culture. Further, the imaging apparatus 10 generates images of the fertilized eggs which are obtained by capturing images of the fertilized eggs by capturing images of the fertilized eggs cultured in the culture medium M1 at predetermined time intervals. The number of captured images of fertilized eggs, an image capturing interval, an image capturing period, a focus position, and the like, may be arbitrarily set. For example, in a case where a culture period is eight days, a total of approximately 600 images of a fertilized egg are captured for one fertilized egg by setting the image capturing interval at 20 minutes.

FIG. 2 schematically illustrates an example of the culture medium M1 in which a plurality of fertilized eggs is cultured. A plurality of wells W1 is provided at the culture medium M1. One fertilized egg E1 is cultured in each well W1. Note that, while FIG. 2 illustrates a case where 7 × 6 = 42 wells W1 are arranged in a matrix within the culture medium M1, the number of the wells W1 and an arrangement state within the culture medium M1 are not particularly limited to this case.

The imaging apparatus 10 generates a plurality of images of fertilized eggs by capturing images of the culture medium M1 in which the plurality of fertilized eggs is cultured in chronological order. The image of the fertilized eggs generated by a single image capturing by the imaging apparatus 10 may include all the fertilized eggs cultured in the culture medium M1 or part of the fertilized eggs. For example, the imaging apparatus 10 may adjust an imaging field of view so as to be able to capture an image of a large number of fertilized eggs in one image capturing, and capture an image of all the fertilized eggs included in the culture medium M1 through one or a plurality of times of image capturing. In a case of the example illustrated in FIG. 2, the imaging apparatus 10 can capture images of all the fertilized eggs included in the culture medium M1 through seven times of image capturing processing by adjusting the imaging field of view so as to be able to capture an image of, for example, six fertilized eggs with each image capturing. The imaging apparatus 10 generates the images of the fertilized eggs as described above and outputs the generated images of the fertilized eggs to the information processing apparatus 20.

### (Information processing apparatus)

The information processing apparatus 20 is an apparatus which analyzes the images of the fertilized eggs captured by the imaging apparatus 10 in chronological order and dynamically controls implementation conditions of image analysis which is to be implemented next time and thereafter on the basis of the analysis result. Further, the information processing apparatus 20 preferably further has a function of determining states of the fertilized eggs on the basis of feature amounts of the fertilized eggs which are feature amounts of the fertilized eggs obtained as a result of image analysis. Still further, the information processing apparatus 20 can display the analysis result of the images of the fertilized eggs captured by the imaging apparatus 10, a determination result regarding the states of the fertilized eggs, and the like, at the display apparatus 30, to present these results to a user. The information processing apparatus 20 includes an apparatus which has a function of implementing image analysis, such as a personal computer (PC) and a large-scale computer on the cloud connected to the Internet.

The information processing apparatus 20 acquires the captured images of the fertilized eggs from the imaging apparatus 10. The information processing apparatus 20 calculates feature amounts of the fertilized eggs which are information representing states of the fertilized eggs from each of a plurality of images of the fertilized eggs by analyzing each of the acquired plurality of images of the fertilized eggs. Further, the information processing apparatus 20 preferably determines the states of the fertilized eggs on the basis of the feature amounts of the fertilized eggs obtained as a result of image analysis. The images of the fertilized eggs captured by the imaging apparatus 10, the result of image analysis and the determination result of the states of the fertilized eggs by the information processing apparatus 20, and the like, are transmitted to the display apparatus 30 and presented to the user.

### (Display apparatus)

The display apparatus 30 is an apparatus which displays the images of the fertilized eggs captured by the imaging apparatus 10, the result of image analysis by the information processing apparatus 20, and the like. The display apparatus 30 includes an image display apparatus such as a liquid crystal display. The display apparatus 30 may display various kinds of information indicating an implementation status of image analysis by the information processing apparatus 20.

The outline of the information processing system 1 according to an embodiment of the present disclosure has been described above. The information processing apparatus 20 included in the information processing system 1 according to an embodiment of the present disclosure is implemented in the following embodiment. Specific configuration examples and processing examples of the information processing apparatus 20 will be described below.

### <<2. Information processing apparatus>>

Hereinafter, the information processing apparatus 20 according to an embodiment of the present disclosure will be explained.

### <2.1. Configuration example>

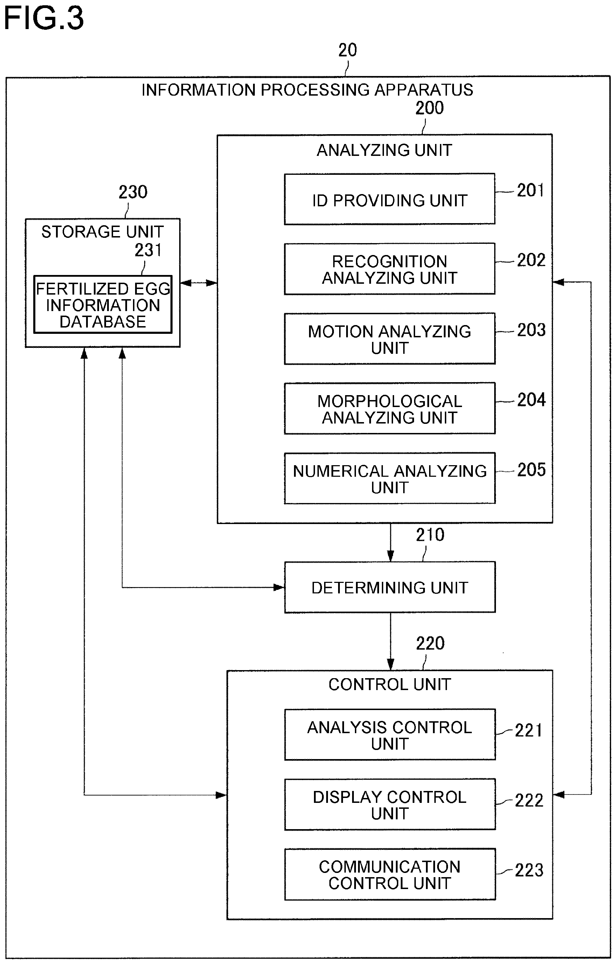

FIG. 3 is a functional block diagram illustrating a functional configuration example of an information processing apparatus 20 according to an embodiment of the present disclosure. As illustrated in FIG. 3, the information processing apparatus 20 includes an analyzing unit 200, a determining unit 210, a control unit 220, and a storage unit 230. Functions of the analyzing unit 200, the determining unit 210 and the control unit 220 are achieved by, for example, a processing circuit having a central processing unit (CPU), a read only memory (ROM), a random access memory (RAM), and the like, provided at the information processing apparatus 20. Further, functions of the storage unit 230 are achieved by a storage apparatus such as a RAM and a storage provided at the information processing apparatus 20. Note that the analyzing unit 200, the determining unit 210, the control unit 220 or the storage unit 230 may be disposed on a large-scale computer on the cloud connected to the Internet. Functions of the respective functional units will be described below.

### (Analyzing unit)

The analyzing unit 200 has a function of analyzing the images of the fertilized eggs captured by the imaging apparatus 10 in chronological order and calculating feature amounts of the fertilized eggs representing the states of the fertilized eggs. Note that the analyzing unit 200 implements image analysis on the basis of implementation conditions of image analysis determined by an analysis control unit 221 which will be described later.

The result of image analysis by the analyzing unit 200 is transmitted to the determining unit 210 and the control unit 220. Further, the result of image analysis by the analyzing unit 200 may be recorded in the storage unit 230. Still further, the result of image analysis by the analyzing unit 200 may be transmitted to the determining unit 210 and the control unit 220 via the storage unit 230.

The analyzing unit 200 includes an ID providing unit 201, a recognition analyzing unit 202, a motion analyzing unit 203, a morphological analyzing unit 204, and a numerical analyzing unit 205 as functional units which analyze the images of the fertilized eggs. The respective functional units included in the analyzing unit 200 implement various kinds of image analysis processing such as provision of identification numbers and calculation of feature amounts of the fertilized eggs on each of the fertilized eggs included in the images of the fertilized eggs. The respective functional units included in the analyzing unit 200 may share the information generated through processing by the respective functional units included in the analyzing unit 200 with each other. Functions of the respective functional units included in the analyzing unit 200 will be described later.

### (Determining unit)

The determining unit 210 has a function of determining the states of the fertilized eggs on the basis of the feature amounts of the fertilized eggs calculated by the analyzing unit 200 analyzing the images of the fertilized eggs.

The determining unit 210 may comprehensively determine the states of the fertilized eggs by comparing the feature amounts of the fertilized eggs with, for example, predetermined thresholds respectively set for the feature amounts of the fertilized eggs, or comparing the feature amounts of the fertilized eggs with temporal change amounts of the feature amounts of the fertilized eggs. Further, the determining unit 210 may comprehensively determine the states of the fertilized eggs on the basis of temporal change of the respective feature amounts of the fertilized eggs.

Further, the determining unit 210 may determine the states of the fertilized eggs using a classifier constructed in advance. The classifier has a function of classifying the states of the fertilized eggs on the basis of input data which is information regarding the fertilized eggs input to the classifier and outputting the classification result as output data. For example, the classifier may classify growth states and quality of the fertilized eggs on the basis of the feature amounts of the fertilized eggs as input data and may output the classification result as output data. The determining unit 210 can input the feature amounts of the fertilized eggs calculated by the analyzing unit 200 to the classifier constructed in advance and can determine output data of the classifier as the states of the fertilized eggs. Further, the classifier can be constructed in advance as a learned model which is created using predetermined machine learning algorithm which receives the feature amounts of the fertilized eggs as input data and outputs the states of the fertilized eggs as output data. In the machine learning algorithm, examples of input data which is information regarding the fertilized eggs input to the classifier by a supervisor (embryologist) in a supervised learning method can include growth stages of the fertilized eggs (for example, respective growth stages of a one-cell stage, a two-cell stage, a three-cell stage, a four-cell stage, an eight-cell stage, a morula stage, an initial blastocyst, a blastocyst stage, an expanded blastocyst stage, a hatched blastocyst stage, a hatched expanded blastocyst stage and a hatching state), a quality condition, for example, the number of blastomeres, symmetric property, a shape and an area of blastomeres, normal cleavage, abnormal cleavage (reverse cleavage, direct cleavage), presence of fragmentations, the number of fragmentations, a ratio of fragmentations, a thickness, a ratio and density of a trophectoderm (TE), presence, a size, a ratio and density of an inner cell mass (ICM), a thickness, circularity and a diameter of a zona pellucida, Veeck classification, Gardner classification, and the like, a first cleavage period, a second cleavage period, a compaction period, a hatching period, the number of pronuclei, the number of polar bodies, presence of dead cells, the number of dead cells, a ratio of dead cells, and the like. Note that input data which is information regarding the fertilized eggs to be input to the classifier by the supervisor (embryologist) is obtained by providing input data to images of the fertilized eggs captured in chronological order, images of the fertilized eggs obtained by performing image processing on the images, or the like. As the predetermined machine learning algorithm, for example, various kinds of publicly known deep neural networks (DNNs) using a neural network such as a recurrent neural network (RNN) and a convolutional neural network (CNN) may be used. Further, as other kinds of machine learning algorithm, various kinds of arbitrary machine learning algorithm which execute a supervised learning method, an unsupervised learning method, a semi-supervised learning method, a reinforcement learning method, or the like, may be used.

The states of the fertilized eggs determined by the determining unit 210 include, for example, growth states of the fertilized eggs, states as to whether quality is good or poor, states as to whether the fertilized eggs are dead or alive, and the like. The result regarding the states of the fertilized eggs determined by the determining unit 210 is transmitted to the control unit 220. Further, the result regarding the states of the fertilized eggs determined by the determining unit 210 may be recorded in the storage unit 230. Still further, the result regarding the states of the fertilized eggs determined by the determining unit 210 may be transmitted to the control unit 220 via the storage unit 230.

### (Control unit)

The control unit 220 has a function of controlling various kinds of processing to be performed by the information processing apparatus 20. For example, the control unit 220 controls the implementation conditions of image analysis, communication with external apparatuses and display of information indicating the implementation status of image analysis. These kinds of control are respectively performed by respective functional units of an analysis control unit 221, a display control unit 222 and a communication control unit 223 provided at the control unit 220. Information generated through processing by the respective functional units included in the control unit 220 is transmitted to the analyzing unit 200. Further, the generated information may be recorded in the storage unit 230 and transmitted to the analyzing unit 200 via the storage unit 230. Further, the respective functional units included in the control unit 220 may share the information to be generated through processing of the respective functional units included in the control unit 220 with each other. Functions of the respective functional units included in the control unit 220 will be described later.

### (Storage unit)

The storage unit 230 has a function of recording the information transmitted to the information processing apparatus 20 and the information generated at the information processing apparatus 20. More specifically, the storage unit 230 can acquire and store the images of the fertilized eggs captured by the imaging apparatus 10. Further, the storage unit 230 may store various kinds of information regarding image analysis such as the feature amounts of the fertilized eggs calculated by the analyzing unit 200, the result of determination by the determining unit 210 and information representing details to be controlled by the control unit 220. The storage unit 230 outputs the information in response to requests from the respective functional units of the analyzing unit 200, the determining unit 210 and the control unit 220.

Further, the storage unit 230 includes a fertilized egg information database 231. In the fertilized egg information database 231, various kinds of information regarding the fertilized eggs such as the feature amounts of the fertilized eggs calculated by the analyzing unit 200 are recorded. Further, in this storage unit 230, evaluation results, and the like, of the fertilized eggs by a user of the information processing system 1 according to the present disclosure may be recorded.

The outline of the analyzing unit 200, the determining unit 210, the control unit 220 and the storage unit 230 provided at the information processing apparatus 20 according to an embodiment of the present disclosure has been described above. Next, functions of the respective functional units respectively provided at the analyzing unit 200 and the control unit 220 will be described.

The respective functional units provided at the analyzing unit 200 will be described first.

### (ID providing unit)

The ID providing unit 201 has a function of providing unique identification numbers to respective fertilized eggs included in the images of the fertilized eggs transmitted from the imaging apparatus 10 to the information processing apparatus 20. The identification number is associated with various kinds of information such as the feature amounts of the fertilized egg to which the identification number is provided. This association enables various kinds of information such as the feature amounts of the fertilized egg on which attention is focused to be linked with the identification number, so that it is possible to collectively manage the information and improve user-friendliness of information management.

### (Recognition analyzing unit)

The recognition analyzing unit 202 has a function of calculating a recognition feature amount which is information regarding an outer shape, a shape, a diameter, an area, a position, or the like, of the fertilized egg from each of a plurality of images of the fertilized egg by analyzing each of the plurality of captured images of the fertilized egg and recognizing a region regarding the fertilized egg within the images of the fertilized egg. Analysis of the images of the fertilized egg, recognition of a specific region regarding the fertilized egg and calculation of a recognition feature amount of the fertilized egg performed by the recognition analyzing unit 202 will be referred to as recognition analysis in the following description.

The recognition analyzing unit 202 analyzes the images of the fertilized egg to recognize a specific region of the fertilized egg included in the images of the fertilized egg. The recognition analyzing unit 202 can recognize various kinds of specific regions regarding the fertilized egg, such as the fertilized egg, an inner cell mass of the fertilized egg or a transparent body of the fertilized egg through an image recognition deep learning technology and an image analysis technology. For example, the recognition analyzing unit 202 can recognize a region of the fertilized egg from the images of the fertilized egg including the fertilized egg and recognize a shape, a diameter, an area, and the like, of the fertilized egg regardless of brightness of background images of the images of the fertilized egg. More specifically, the recognition analyzing unit 202 can extract features such as an outline of the fertilized egg, or the like, from the information included in the images of the fertilized egg and can recognize these regions regarding the fertilized egg using a pattern matching technology of matching the features with features of targets prepared in advance. Further, the recognition analyzing unit 202 may recognize these regions regarding the fertilized egg using an image analysis method using various kinds of publicly known algorithm such as machine learning using a deep neural network.

Further, the recognition analyzing unit 202 calculates various kinds of recognition feature amounts on the basis of the recognized various kinds of regions of the fertilized egg.

The cell is a fertilized egg, and the recognition feature amount includes, for example, an area of the fertilized egg, a diameter of the fertilized egg, circularity of the fertilized egg, ellipticity of the fertilized egg, a position of center of gravity of the fertilized egg, a central position of the fertilized egg, an area of an inner cell mass of the fertilized egg, a diameter of the inner cell mass of the fertilized egg, circularity of the inner cell mass of the fertilized egg, ellipticity of the inner cell mass of the fertilized egg, a position of center of gravity of the inner cell mass of the fertilized egg, a central position of the inner cell mass of the fertilized egg, an area of a zona pellucida of the fertilized egg, a diameter of the zona pellucida of the fertilized egg, circularity of the zona pellucida of the fertilized egg, ellipticity of the zona pellucida of the fertilized egg, a position of center of gravity of the zona pellucida of the fertilized egg, a central position of the zona pellucida of the fertilized egg, a number of pronuclei of the fertilized egg, an area of the pronuclei of the fertilized egg, a number of polar bodies of the fertilized egg, an area of the polar bodies of the fertilized egg, a number of fragmentations of the fertilized egg, or an area of the fragmentations of the fertilized egg, or the like.

### (Motion analyzing unit)

The motion analyzing unit 203 has a function of analyzing a plurality of images of the fertilized egg captured in chronological order to calculate a motion feature amount which is information regarding motion of the fertilized egg from each of the plurality of images of the fertilized egg. Analysis of the images of the fertilized egg and calculation of the motion feature amount performed by the motion analyzing unit 203 will be referred to as motion analysis in the following description.

The cell is a fertilized egg, and the motion feature amount includes, for example, a motion amount of the whole fertilized egg, a motion direction of the whole fertilized egg, a motion amount of blastomeres inside the fertilized egg, a motion amount of an inner cell mass within cells of a blastocyst inside the fertilized egg, or a motion direction of the inner cell mass (ICM) within cells of the blastocyst inside the fertilized egg, or the like. Here, the motion amount means a magnitude of motion per unit time (unit frame). Further, the motion direction means a direction of motion per unit time (unit frame). These motion amount and motion direction may correspond to, for example, a magnitude and a direction of a specified motion vector.

The motion vector can be specified using, for example, a block matching method. The block matching method is a method in which an image is divided into a plurality of blocks including, for example, N × N pixels, a block which is the closest to a block within a region of interest is searched for from frames before and after the frame, and the motion vector is calculated on the basis of a difference between the blocks.

Various kinds of motion feature amounts can be calculated on the basis of the motion vector. More specifically, the motion analyzing unit 203 may calculate an average value of a plurality of motion vectors specified in a region of the fertilized egg as the motion feature amount. For example, the motion analyzing unit 203 can calculate a motion amount of the whole fertilized egg as an average value of the magnitudes of the plurality of motion vectors specified in a region of the whole fertilized egg.

Note that a method for specifying the above-described motion vector is not limited to the above-described block matching method, and the motion vector may be specified using various kinds of publicly known algorithm.

### (Morphological analyzing unit)

The morphological analyzing unit 204 has a function of analyzing each of the plurality of captured images of the fertilized egg to calculate a morphological feature amount which is information representing features regarding morphology of the fertilized egg from each of the plurality of images of the fertilized egg. Analysis of the images of the fertilized egg and calculation of the morphological feature amount performed by the morphological analyzing unit 204 will be referred to as morphological analysis in the following description.

The morphological analyzing unit 204 may calculate the morphological feature amount using an arbitrary image analysis method using publicly known algorithm such as machine learning using deep learning. The morphological analyzing unit 204 can calculate the morphological feature amount of the fertilized egg using various kinds of publicly known image analysis technology. For example, the morphological analyzing unit 204 may calculate the morphological feature amount using algorithm which executes a supervised learning method based on training data of observation evaluation of the fertilized egg by an embryologist. The observation evaluation by the embryologist may include a growth stage and a quality condition of the fertilized egg. Further, the morphological analyzing unit 204 can use a pattern matching technology of extracting features such as an outline of the fertilized egg from the images of the fertilized egg and matching the features with features of a target prepared in advance.

The morphological feature amount includes, for example, a growth state representing a stage of growth of the fertilized egg or a quality condition representing quality of the fertilized egg. The growth state of the fertilized egg includes an early-stage fertile ovum of one cell, two cells, four cells, eight cells, and the like, a morula, an initial blastocyst, a blastocyst, an expanded blastocyst, and the like. Further, the quality condition of the fertilized egg includes the number of blastomeres of the fertilized egg, the number of fragments, the number of polar bodies, the number of pronuclei, the number of lipid granules, Veeck classification, Gardner classification, presence of direct cleavage of the fertilized egg, presence of reverse cleavage of the fertilized egg, and the like.

Here, the above-described direct cleavage is one type of abnormal cleavage in which the fertilized egg is non-equally divided, for example, divided into three cells from one cell, divided into six cells from two cells, or the like. Further, the above-described reverse cleavage is one type of abnormal cleavage in which the cleavage stage of the fertilized egg reversely proceeds, for example, from three cells to two cells or from eight cells to six cells.

### (Numerical analyzing unit)

The numerical analyzing unit 205 has a function of analyzing each of the plurality of captured images of the fertilized egg to calculate a numerical feature amount which is a numerical value representing a state of the fertilized egg from each of the plurality of images of the fertilized egg. Analysis of the images of the fertilized egg and calculation of the recognition feature amount performed by the numerical analyzing unit 205 will be referred to as numerical analysis in the following description.

The cell is a fertilized egg, and the numerical feature amount includes, for example, an amount of contraction of an area of the fertilized egg, a ratio of contraction of the area of the fertilized egg, contraction speed of the area of the fertilized egg, a contraction period of the area of the fertilized egg, a cumulative amount of movement of the fertilized egg, a number of times of contraction of a total area of the fertilized egg, an amount of contraction of an area of inner cells of the fertilized egg, a ratio of contraction of the area of the inner cells of the fertilized egg, contraction speed of the area of the inner cells of the fertilized egg, a contraction period of the area of the inner cells of the fertilized egg, a number of times of contraction of the area of the inner cells of the fertilized egg, a cleavage period, abnormal cleavage or a compaction period, or the like. These numerical feature amounts may be calculated by the numerical analyzing unit 205 on the basis of the morphological feature amount, the motion feature amount and the recognition feature amount calculated by the recognition analyzing unit 202, the motion analyzing unit 203 and the morphological analyzing unit 204.

The respective functional units provided at the analyzing unit 200 have been described above. Next, functions of the respective functional units provided at the control unit 220 will be described.

### (Analysis control unit)

The analysis control unit 221 has a function of dynamically controlling implementation conditions of image analysis to be implemented by the analyzing unit 200 on the basis of the feature amounts of the fertilized egg calculated by the analyzing unit 200. The analysis control unit 221 may dynamically control the implementation conditions of image analysis on the basis of temporal change of the feature amounts of the fertilized egg. Further, the analysis control unit 221 may determine the implementation conditions of image analysis to be implemented by the analyzing unit 200 on the basis of growth and quality of the fertilized egg determined by the determining unit 210.

For example, the analysis control unit 221 can determine continuation, stop, interruption, restart, or the like, of image analysis by the analyzing unit 200 next time and thereafter for the fertilized egg on the basis of the feature amounts of the fertilized egg on which attention is focused. Here, continuation of image analysis means continuously implementing image analysis on each frame thereafter. Further, stop of image analysis means not implementing image analysis thereafter. Note that even a fertilized egg for which image analysis has been stopped can be subjected to image analysis by image analysis being restarted, or the like, by the analysis control unit 221. Further, interruption of image analysis means implementing image analysis while a predetermined time interval during which image analysis is not implemented being occasionally provided. Note that the predetermined time interval may be a fixed time interval regardless of time or may be made longer as time elapses. Further, restart of image analysis means implementing image analysis again for a fertilized egg for which image analysis has been stopped.

The analysis control unit 221 transmits the implementation conditions of image analysis determined by the analysis control unit 221 to the analyzing unit 200. The analyzing unit 200 implements image analysis in accordance with the implementation conditions of image analysis determined by the analysis control unit 221. In this manner, the analysis control unit 221 controls the implementation conditions of image analysis to be implemented by the analyzing unit 200 next time and thereafter. Further, the implementation conditions of image analysis are dynamically controlled by such control being performed by the analysis control unit 221 for each predetermined period (frame). As a result of the conditions of image analysis which is processing involving various kinds of cost being appropriately controlled in this manner, cost required for image analysis is reduced.

The analysis control unit 221 may determine various kinds of implementation conditions such as continuation, stop, interruption and restart of calculation by the analyzing unit 200 for part of a plurality of feature amounts of the fertilized egg on which attention is focused. Further, the analysis control unit 221 may determine to reduce the number of feature amounts of the fertilized egg on which attention is focused, to be calculated by the analyzing unit 200 as time elapses. By this means, the analyzing unit 200 reduces the number of feature amounts of the fertilized egg to be calculated as time elapses. Calculation of the feature amounts of the fertilized egg by the analyzing unit 200 involves cost for calculation, and the like, by a computer which constitutes the analyzing unit 200. Typically, cost for calculation, or the like, by the computer becomes higher as the number of feature amounts of the fertilized egg to be calculated becomes larger. In other words, as the number of feature amounts of the fertilized egg to be calculated by the analyzing unit 200 becomes smaller, cost required for image analysis becomes lower. Therefore, as a result of the feature amounts of the fertilized egg to be calculated by the analyzing unit 200 being reduced as time elapses, cost required for image analysis becomes gradually lower.

The analysis control unit 221 may determine various kinds of implementation conditions of image analysis such as continuation, stop, interruption and restart of part of methods for implementing image analysis for the fertilized egg on which attention is focused. Further, the analysis control unit 221 may reduce the number of analysis methods to be used in image analysis as time elapses for the fertilized egg on which attention is focused. Note that the analysis methods described here include recognition analysis, motion analysis, morphological analysis and numerical analysis.

Further, the analysis control unit 221 may determine to reduce the number of analysis methods to be used in image analysis after the number of feature amounts of the fertilized egg to be calculated is reduced for the fertilized egg on which attention is focused. For example, the analysis control unit 221 reduces the number of motion feature amounts to be calculated through motion analysis which is one of the analysis methods for the fertilized egg on which attention is focused. Then, the analysis control unit 221 can determine to stop, interrupt, or the like, motion analysis when the number of motion feature amounts to be calculated falls below a predetermined number for the fertilized egg.

Note that the analysis control unit 221 may determine to reduce the number of analysis methods to be used in image analysis in a case where calculation of a predetermined feature amount of the fertilized egg is stopped for the fertilized egg on which attention is focused.

Further, the analysis control unit 221 can change the implementation conditions of image analysis which has been determined once, for each frame on the basis of the feature amounts of the fertilized egg. For example, in a case where the analysis control unit 221 determines to stop calculation of part of a plurality of feature amounts of the fertilized egg on which attention is focused, the analysis control unit 221 can determine to restart calculation of the feature amounts of the fertilized egg which has been stopped, on the basis of the feature amounts of the fertilized egg for which calculation is continued. By this means, the analyzing unit 200 restarts calculation of the feature amounts of the fertilized egg which has been stopped.

The implementation conditions of image analysis determined by the analysis control unit 221 may be recorded in the storage unit 230. The implementation conditions of image analysis recorded in the storage unit 230 can be output to the analyzing unit 200 from the storage unit 230 in response to a request from the analyzing unit 200.

### (Display control unit)

The display control unit 222 has a function of controlling display of the information acquired by the information processing apparatus 20 at the display apparatus 30. For example, the display control unit 222 controls the communication control unit 223 which will be described later so as to display various kinds of information regarding image analysis such as the feature amounts of the fertilized egg calculated by the analyzing unit 200 and information indicating the implementation conditions of image analysis determined by the analysis control unit 221 at the display apparatus 30.

### (Communication control unit)

The communication control unit 223 has a function of controlling communication from the information processing apparatus 20 to various kinds of external apparatuses. As a result of the communication control unit 223 controlling a communication device (which is not illustrated in FIG. 2) having a function of communicating with external apparatuses in a wired or wireless manner, communication from the information processing apparatus 20 to various kinds of external apparatuses is performed. The communication device may be included in the information processing apparatus 20. Further, the communication control unit 223 may be connected to a large-scale computer such as an external cloud through a communication line, for example, an Internet connection.

For example, the communication control unit 223 controls the communication device to communicate with the imaging apparatus 10. More specifically, the communication control unit 223 controls the communication device to acquire the images of the fertilized egg captured by the imaging apparatus 10. Further, the communication control unit 223 controls the communication device to communicate with the display apparatus 30. For example, the communication control unit 223 controls the communication device to transmit various kinds of information regarding image analysis such as the feature amounts of the fertilized egg calculated by the analyzing unit 200 and information indicating the implementation conditions of image analysis determined by the analysis control unit 221 to the display apparatus 30 through control by the display control unit 222. By this means, various kinds of information regarding image analysis are transmitted from the communication device to the display apparatus 30 and are displayed at the display apparatus.

### <2.2. Processing example>

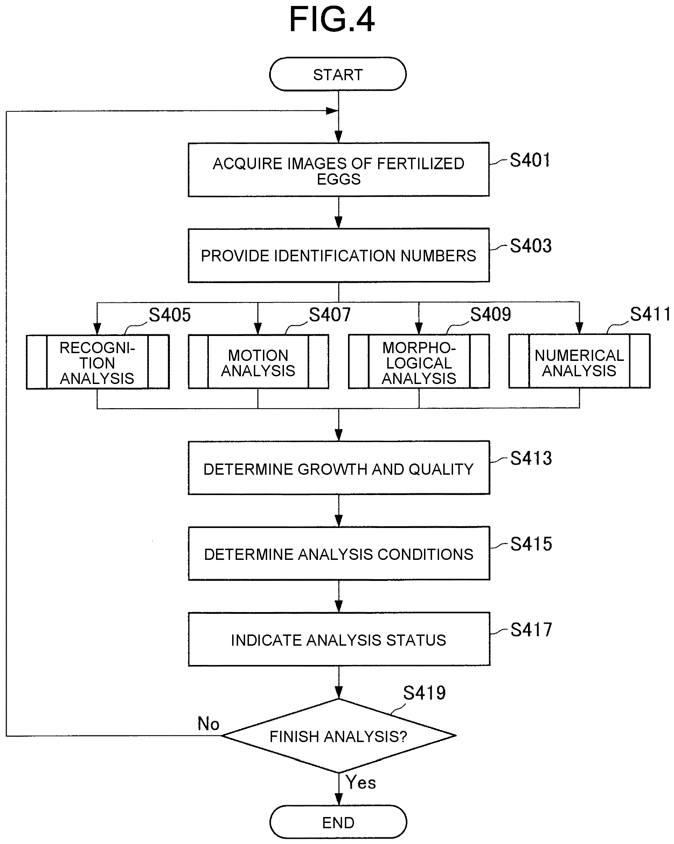

The configuration and the functions of the information processing apparatus 20 according to an embodiment of the present disclosure have been described above. Next, an example of processing to be performed by the information processing apparatus 20 according to an embodiment of the present disclosure will be described using FIG. 4 to FIG. 8.

FIG. 4 is a flowchart illustrating an example of processing to be performed by the information processing apparatus 20 according to an embodiment of the present disclosure. The flowchart illustrated in FIG. 4 is an example illustrating flow of processing in which the information processing apparatus 20 acquires images of the fertilized eggs from the imaging apparatus 10, calculates feature amounts of the fertilized eggs from the images of the fertilized eggs, determines implementation conditions of image analysis next time and thereafter on the basis of the calculated feature amounts of the fertilized eggs, and transmits information indicating an implementation status of image analysis to the display apparatus 30.

First, the storage unit 230 acquires a plurality of captured images of the fertilized eggs from the imaging apparatus 10 (step S401). The acquired images of the fertilized eggs are recorded in the storage unit 230 and further transmitted to the analyzing unit 200.

The ID providing unit 201 then provides unique identification numbers to the respective fertilized eggs included in the acquired images of the fertilized eggs (step S403).

The recognition analyzing unit 202, the motion analyzing unit 203, the morphological analyzing unit 204 or the numerical analyzing unit 205 then respectively performs recognition analysis processing, motion analysis processing, morphological analysis processing or numerical analysis processing in parallel on each of the acquired images of the fertilized eggs (step S405, S407, S409 or S411). These kinds of analysis processing are performed for each fertilized egg included in the images of the fertilized eggs. In other words, in a case where there are N fertilized eggs in the captured images, these kinds of analysis processing are performed N times each. These recognition analysis processing, motion analysis processing, morphological analysis processing and numerical analysis processing will be described in detail later.

The determining unit 210 then determines whether each fertilized egg is well grown or poorly grown and whether quality is good or poor on the basis of feature amounts of the fertilized eggs calculated through the recognition analysis processing, the motion analysis processing, the morphological analysis processing or the numerical analysis processing (step S413).

The analysis control unit 221 determines implementation conditions of image analysis to be implemented by the analyzing unit 200 on the basis of the growth and the quality of the fertilized eggs determined by the determining unit 210 (step S415). For example, the analysis control unit 221 can determine to continue image analysis by the analyzing unit 200 for the fertilized eggs whose quality is determined as good by the determining unit 210. Further, the analysis control unit 221 may determine to stop or interrupt image analysis for the fertilized eggs whose quality is determined as poor by the determining unit 210.

The implementation conditions of image analysis to be implemented by the analyzing unit 200 determined by the analysis control unit 221 are transmitted to the analyzing unit 200. Note that the implementation conditions of image analysis to be implemented by the analyzing unit 200 determined by the analysis control unit 221 may be recorded in the storage unit 230.

The display control unit 222 then controls the communication control unit 223 so as to display various kinds of information regarding image analysis such as the feature amounts of the fertilized eggs calculated through image analysis and information indicating an implementation status of image analysis at the display apparatus 30 (step S417). Various kinds of information regarding image analysis are displayed at the display apparatus 30.

The analysis control unit 221 then determines whether or not to finish image analysis by the analyzing unit 200 (step S419). The analysis control unit 221 may determine to finish image analysis, for example, when a predetermined period elapses and culture of the fertilized eggs is finished. In a case where the analysis control unit 221 determines to finish image analysis (step S419: Yes), the control flow in FIG. 4 is finished. In a case where the analysis control unit 221 determines not to finish image analysis (step S419: No), the processing returns to step S401. Thereafter, the processing from step S401 to step S419 is repeatedly performed until the analysis control unit 221 determines to finish image analysis in step S419.

The outline of the processing to be performed by the information processing apparatus 20 according to an embodiment of the present disclosure has been described above. The recognition analysis processing (step S405), the motion analysis processing (step S407), the morphological analysis processing (step S409) and the numerical analysis processing (step S411) which are part of the processing described here will be described in detail below.

### [Recognition analysis processing]

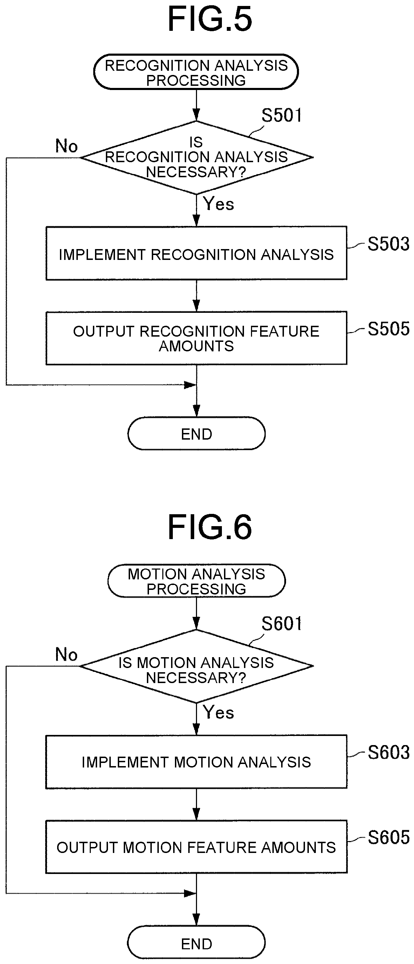

First, the recognition analysis processing to be performed in step S405 will be described. FIG. 5 is a flowchart for explaining flow of recognition analysis processing.

First, the recognition analyzing unit 202 determines whether or not it is necessary to perform recognition analysis on the basis of the implementation conditions of image analysis determined by the analysis control unit 221 (step S501). For example, the recognition analyzing unit 202 can determine that it is necessary to perform recognition analysis for the fertilized eggs for which it is determined by the analysis control unit 221 to continue recognition analysis. Meanwhile, the recognition analyzing unit 202 can determine that it is not necessary to perform recognition analysis for the fertilized eggs for which it is determined by the analysis control unit 221 to stop recognition analysis. In a case where the recognition analyzing unit 202 determines that it is necessary to perform recognition analysis (step S501: Yes), the processing proceeds to step S503. Meanwhile, in a case where the recognition analyzing unit 202 determines that it is not necessary to perform recognition analysis (step S501: No), the recognition analysis processing is finished.

The recognition analyzing unit 202 then implements recognition analysis on the fertilized eggs included in the images of the fertilized eggs on the basis of the implementation conditions of image analysis determined by the analysis control unit 221 (step S503). For example, the recognition analyzing unit 202 calculates part of recognition feature amounts for the fertilized eggs for which it is determined to calculate part of the recognition feature amounts. Alternatively, the recognition analyzing unit 202 only recognizes regions of the whole fertilized eggs for the fertilized eggs for which it is determined to only recognize the regions of the whole fertilized eggs without calculating the recognition feature amounts.

The recognition analyzing unit 202 then outputs the recognition feature amounts calculated by the recognition analyzing unit 202 (step S505). The output recognition feature amounts are transmitted to the determining unit 210 and the control unit 220. Further, the output recognition feature amounts may be stored in the storage unit 230.

The recognition analysis processing has been described above. Next, the motion analysis processing will be described.

### [Motion analysis processing]

First, the motion analysis processing to be performed in step S407 will be described. FIG. 6 is a flowchart for explaining flow of motion analysis processing.

First, the motion analyzing unit 203 determines whether or not it is necessary to perform motion analysis on the basis of the implementation conditions of image analysis determined by the analysis control unit 221 (step S601). For example, the motion analyzing unit 203 can determine that it is necessary to perform motion analysis for the fertilized eggs for which it is determined by the analysis control unit 221 to continue recognition analysis. Meanwhile, the motion analyzing unit 203 can determine that it is not necessary to perform motion analysis for the fertilized eggs for which it is determined by the analysis control unit 221 to stop motion analysis. In a case where the motion analyzing unit 203 determines that it is necessary to perform motion analysis (step S601: Yes), the processing proceeds to step S603. Meanwhile, in a case where the motion analyzing unit 203 determines that it is not necessary to perform motion analysis (step S601: No), the motion analysis processing is finished.

The motion analyzing unit 203 then implements motion analysis on the fertilized eggs included in the images of the fertilized eggs on the basis of the implementation conditions of image analysis determined by the analysis control unit 221 (step S603). For example, the motion analyzing unit 203 calculates part of motion feature amounts for the fertilized eggs for which it is determined to calculate part of the motion feature amounts.

The motion analyzing unit 203 then outputs the motion feature amounts calculated by the motion analyzing unit 203 (step S605). The output motion feature amounts are transmitted to the determining unit 210 or the control unit 220. Further, the output motion feature amounts may be stored in the storage unit 230.

The motion analysis processing has been described above. Next, the morphological analysis processing will be described.

### [Morphological analysis processing]

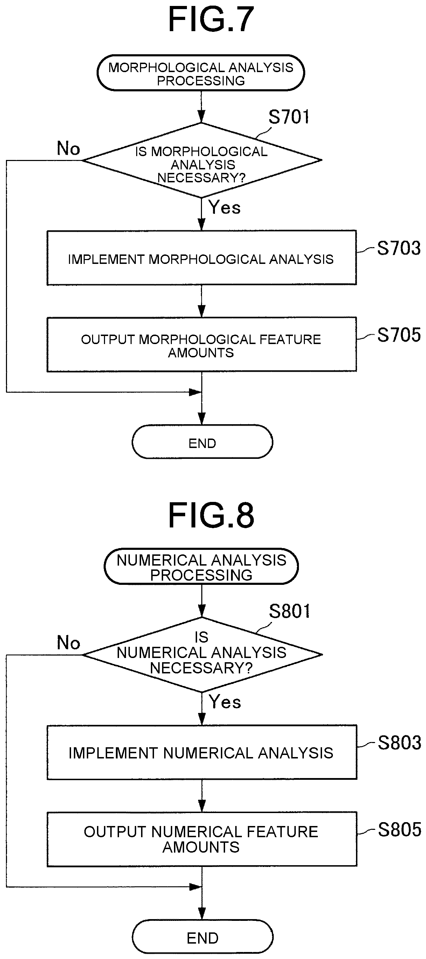

First, the morphological analysis processing to be performed in step S409 will be described. FIG. 7 is a flowchart for explaining flow of morphological analysis processing.

First, the morphological analyzing unit 204 determines whether or not it is necessary to perform morphological analysis on the basis of the implementation conditions of image analysis determined by the analysis control unit 221 (step S701). For example, the morphological analyzing unit 204 can determine that it is necessary to perform morphological analysis for the fertilized eggs for which it is determined by the analysis control unit 221 to continue morphological analysis. Meanwhile, the morphological analyzing unit 204 can determine that it is not necessary to perform morphological analysis for the fertilized eggs for which it is determined by the analysis control unit 221 to stop morphological analysis. In a case where the morphological analyzing unit 204 determines that it is necessary to perform morphological analysis (step S701: Yes), the processing proceeds to step S703. Meanwhile, in a case where the morphological analyzing unit 204 determines that it is not necessary to perform morphological analysis (step S701: No), the morphological analysis processing is finished.

The morphological analyzing unit 204 then implements morphological analysis on the fertilized eggs included in the images of the fertilized eggs on the basis of the implementation conditions of image analysis determined by the analysis control unit 221 (step S703). For example, the morphological analyzing unit 204 calculates part of morphological feature amounts for the fertilized eggs for which it is determined to calculate part of the morphological feature amounts.

The morphological analyzing unit 204 then outputs the morphological feature amounts calculated by the recognition analyzing unit 202 (step S705). The output morphological feature amounts are transmitted to the determining unit 210 or the control unit 220. Further, the output morphological feature amounts may be stored in the storage unit 230.

The morphological analysis processing has been described above. Next, the numerical analysis processing will be described.

### [Numerical analysis processing]

First, the numerical analysis processing to be performed in step S411 will be described. FIG. 8 is a flowchart for explaining flow of numerical analysis processing.

First, the numerical analyzing unit 205 determines whether or not it is necessary to perform numerical analysis on the basis of the implementation conditions of image analysis determined by the analysis control unit 221 (step S801). For example, the numerical analyzing unit 205 can determine that it is necessary to perform numerical analysis for the fertilized eggs for which it is determined by the analysis control unit 221 to continue numerical analysis. Meanwhile, the numerical analyzing unit 205 can determine that it is not necessary to perform numerical analysis for the fertilized eggs for which it is determined by the analysis control unit 221 to stop numerical analysis. In a case where the numerical analyzing unit 205 determines that it is necessary to perform numerical analysis (step S801: Yes), the processing proceeds to step S803. Meanwhile, in a case where the numerical analyzing unit 205 determines that it is not necessary to perform numerical analysis (step S801: No), the numerical analysis processing is finished.

The numerical analyzing unit 205 then implements numerical analysis on the fertilized eggs included in the images of the fertilized eggs on the basis of the implementation conditions of image analysis determined by the analysis control unit 221 (step S803). For example, the numerical analyzing unit 205 calculates part of numerical feature amounts for the fertilized eggs for which it is determined to calculate part of the numerical feature amounts.

The numerical analyzing unit 205 then outputs the numerical feature amounts calculated by the numerical analyzing unit 205 (step S805). The output numerical feature amounts are transmitted to the determining unit 210 or the control unit 220. Further, the output numerical feature amounts may be stored in the storage unit 230.

The numerical analyzing processing has been described above.

The respective kinds of analysis processing to be performed from step S405 to S411 have been described above. Next, processing of displaying an analysis status in step S417 will be described using an example where information indicating the implementation status of image analysis is displayed at the display apparatus 30.

### [Display of analysis status]

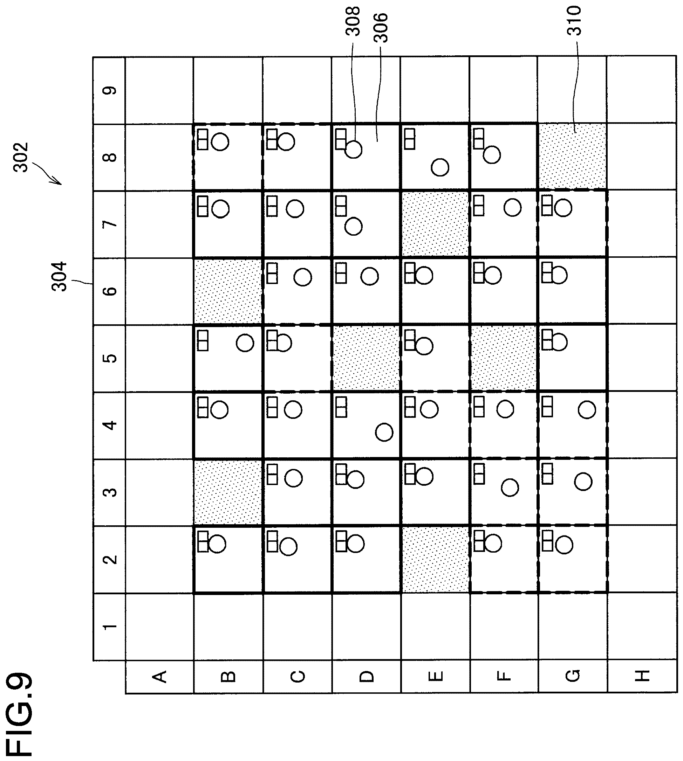

FIG. 9 illustrates an example of a screen of a display apparatus 30 on which information indicating an implementation status of image analysis is displayed. A screen 302 is displayed at the display apparatus 30. The screen 302 includes a display unit 304 which indicates the implementation status of image analysis of the fertilized eggs. As illustrated in FIG. 9, the display unit 304 is divided into sections from row A to row H and from column 1 to column 9. The respective sections correspond to wells W1 in the culture medium M1 in FIG. 2. Further, each well W1 corresponds to one fertilized egg. Therefore, each section corresponds to one fertilized egg. Note that a method for displaying information indicating the implementation status of image analysis of the fertilized eggs is not limited to the example in FIG. 9.

For example, a fertilized egg 308 for which image analysis is implemented is displayed in a section 306 of row D and column 8. Meanwhile, in an embodiment of the present disclosure, the fertilized eggs for which image analysis is not implemented are not displayed at the display apparatus 30. For example, a case will be described where image analysis of a fertilized egg corresponding to a section 310 of row G and column 8 is stopped. In an embodiment of the present disclosure, the sections corresponding to the fertilized eggs for which image analysis is not implemented are, for example, painted with gray. Here, image analysis is not implemented for fertilized eggs with poor quality. Therefore, a user such as an embryologist can distinguish fertilized eggs with poor quality by confirming the fertilized eggs painted with gray at the display unit 304 with reference to the display unit 304 at which the screen 302 is displayed. Further, it is possible to shorten a period for confirming fertilized eggs with poor quality.

Here, an example has been described where sections corresponding to fertilized eggs for which image analysis is stopped are painted with gray. As other examples, it is also possible to blink sections corresponding to fertilized eggs for which image analysis is interrupted. Further, in a case where calculation of part of the feature amounts of the fertilized eggs is stopped or interrupted, marks corresponding to the feature amounts of the fertilized eggs for which calculation is stopped may be displayed at the display unit 304. Still further, in a case where part of the analysis methods to be used in image analysis is stopped or interrupted, marks corresponding to the analysis methods which are stopped or interrupted may be displayed at the display unit 304.

The method for displaying the implementation status of image analysis to be implemented by the information processing apparatus 20 according to an embodiment of the present disclosure has been described above.

The outline of the processing to be performed by the information processing apparatus 20 according to an embodiment of the present disclosure has been described above.

Note that respective steps in the processing of the information processing apparatus 20 in the present specification do not necessarily have to be performed in chronological order in order described in the flowchart. For example, the respective steps in the processing of the information processing apparatus 20 may be performed in order different from the order described in the flowchart or may be performed in parallel. For example, while, in an embodiment of the present disclosure, the recognition analysis processing (step S405), the motion analysis processing (step S407), the morphological analysis processing (step S407) and the numerical analysis processing (step S409) are performed in parallel, all of these kinds of analysis processing may be performed in series or part of these kinds of analysis processing may be performed in series. Further, while, in an embodiment of the present disclosure, determination of growth and quality of the fertilized eggs by the determining unit 210 (step S413) and determination of the implementation conditions of image analysis by the analysis control unit 221 (step S415) are performed in series, these kinds of processing may be performed in parallel.

The configuration example and the processing example of the information processing apparatus 20 according to an embodiment of the present disclosure have been described above.

As described above, the information processing apparatus 20 according to an embodiment of the present disclosure includes the analyzing unit 200 configured to analyze a plurality of images obtained by capturing images of an observation target including a cell having ability to divide in chronological order, to calculate feature amounts representing a state of the cell, and the control unit 220 configured to dynamically control implementation conditions of the analysis on the basis of the calculated feature amounts. Note that the "observation target including a cell having ability to divide" may be, for example, a fertilized egg which is held in a well provided at the culture medium M1. Further, "dynamically control implementation conditions of the analysis" may be, for example, the analysis control unit 221 determining implementation conditions of image analysis to be implemented by the analyzing unit 200 every time the control flow illustrated in FIG. 4 is repeated. The analysis control unit 221 can thereby dynamically control the implementation conditions of image analysis to be implemented by the analyzing unit 200. This will be described in detail below using specific examples.

The processing to be performed by the information processing apparatus 20 will be described more specifically below using first to seventh specific examples. Particularly, a method in which the determining unit 210 determines growth and quality of the fertilized eggs in step S413, and details of the implementation conditions of image analysis to be determined by the analysis control unit 221 in step S415 will be specifically described.

### <2.2.1. First specific example>

In the first specific example, in step S413, the determining unit 210 determines growth and quality of the fertilized eggs on the basis of areas of the fertilized eggs which are one of the recognition feature amounts. More specifically, the determining unit 210 determines the fertilized eggs whose areas change little over time, as the fertilized eggs with poor quality. Further, in the first specific example, in step S415, the analysis control unit 221 determines to stop image analysis by the respective functional units of the analyzing unit 200 for the fertilized eggs whose growth and quality are determined as poor. The processing to be performed by the information processing apparatus 20 in the first specific example will be described below using the flowchart in FIG. 4.

First, the storage unit 230 acquires a plurality of images of the fertilized eggs captured in chronological order from the imaging apparatus 10 (step S401). The images of the fertilized eggs acquired by the storage unit 230 are transmitted to the analyzing unit 200.

The ID providing unit 201 then provides unique identification numbers to the respective fertilized eggs included in the images of the fertilized eggs (step S403).

Then, the recognition analysis processing (step S405), the motion analysis processing (step S407), the morphological analysis processing (step S409) and the numerical analysis processing (processing step S411) are performed in parallel. As a result of these kinds of analysis processing being performed, the recognition feature amounts, the motion feature amounts, the morphological feature amounts and the numerical feature amounts of the respective fertilized eggs included in the images of the fertilized eggs are calculated by the analyzing unit 200.

The determining unit 210 then determines growth and quality of the respective fertilized eggs on the basis of the areas of the fertilized eggs which are one of the recognition feature amounts calculated by the recognition analyzing unit 202 (step S413).

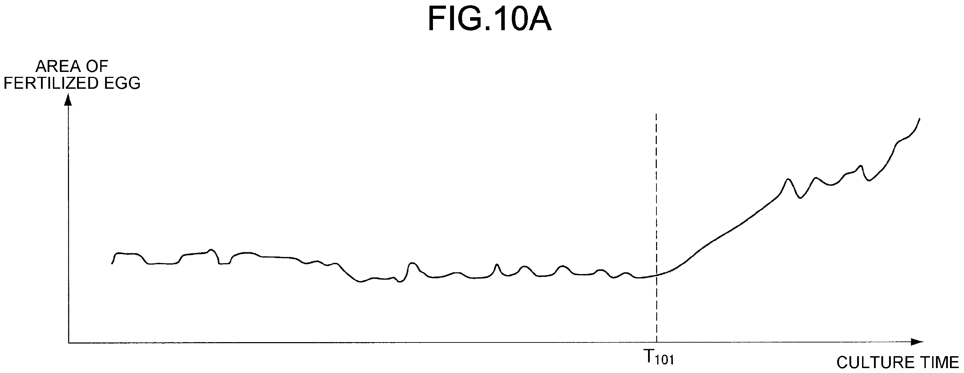

Here, a difference in temporal change of an area between a fertilized egg which is well grown and which has good quality and a fertilized egg which is poorly grown and which has poor quality will be described. FIG. 10A is a graph illustrating an example of relationship between an area of a fertilized egg with good quality and culture time. As illustrated in FIG. 10A, the area of the fertilized egg with good quality fluctuates as the culture time elapses. For example, an area of a fertilized egg largely increases at and after culture time T₁₀₁ in a blastocyst stage and subsequent stages.

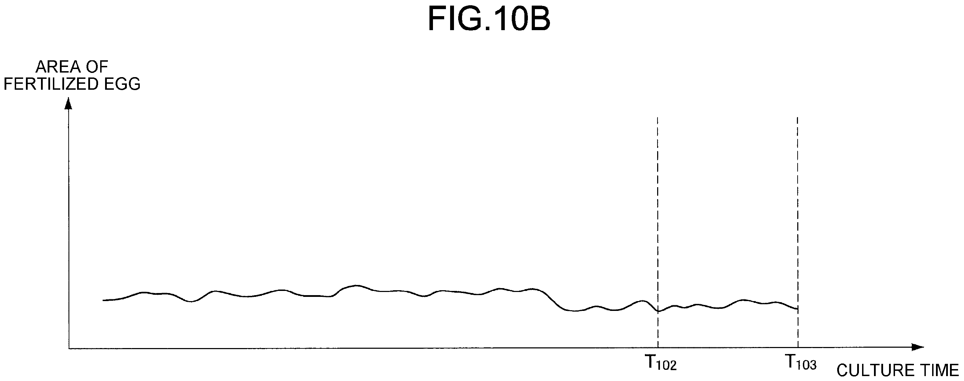

FIG. 10B is a graph illustrating an example of a result obtained by an information processing apparatus 20 in a first specific example calculating an area of a fertilized egg with poor quality. Note that the area is not plotted after culture time T₁₀₃ because image analysis by the analyzing unit 200 is stopped after the culture time T₁₀₃, and thus, calculation of the area of the fertilized egg which is one of the recognition feature amount is stopped. There is a case where temporal change of the area of the fertilized egg with poor quality is small. For example, in the example in FIG. 10B, temporal change of the area of the fertilized egg is small from culture time T₁₀₂ to the culture time T₁₀₃.

In the first specific example, the determining unit 210 determines the fertilized eggs for which temporal change of the areas is small as the fertilized eggs which are poorly grown and which have poor quality. In the example illustrated in FIG. 10B, temporal change of the area of the fertilized egg is small from the culture time T₁₀₂ to the culture time T₁₀₃. The determining unit 210 determines the fertilized eggs which exhibit such temporal change of areas as the fertilized eggs which are poorly grown and which have poor quality at the culture time T₁₀₃. In this event, the determining unit 210 transmits information of the fertilized eggs determined as the fertilized eggs with poor quality to the control unit 220. Further, the determining unit 210 records the information of the fertilized eggs determined as the fertilized eggs with poor quality in the storage unit 230.

The analysis control unit 221 then determines the implementation conditions of image analysis to be implemented by the analyzing unit 200 on the basis of the growth and the quality of the fertilized eggs determined by the determining unit 210 (step S415). Here, description will be provided while attention is focused on a first fertilized egg which is determined as a fertilized egg with poor quality at the culture time T₁₀₃ as described above. In the first specific example, the analysis control unit 221 determines to stop image analysis by the respective functional units of the analyzing unit 200 for the fertilized egg determined as the fertilized egg which is poorly grown and which has poor quality. Note that the analysis control unit 221 can determine to continue image analysis by the respective functions of the analyzing unit 200 for the fertilized eggs which are not determined as the fertilized eggs with poor quality. Here, the determining unit 210 determines the first fertilized egg as the fertilized egg which is poorly grown and which has poor quality at the culture time T₁₀₃. Thus, the analysis control unit 221 determines to stop image analysis by the respective functional units of the analyzing unit 200 for the first fertilized egg. Calculation of the area of the first fertilized egg is thereby stopped after the culture time T₁₀₃ as illustrated in FIG. 10B.

The display control unit 222 then performs control to transmit information indicating an implementation status of image analysis to the display apparatus 30 (step S417). The information indicating the implementation status of image analysis is thereby displayed at the display apparatus 30.

Note that, in the first specific example, information indicating that image analysis of the first fertilized egg is stopped is displayed at the display apparatus 30. For example, a section corresponding to the first fertilized egg can be displayed with gray at the display apparatus 30 as illustrated in the example in FIG. 9. A user such as an embryologist can know from this display that the first fertilized egg is poorly grown and has poor quality.

The analysis control unit 221 then determines whether or not to finish image analysis of the fertilized eggs (step S419). In a case where the analysis control unit 221 determines to finish image analysis of the fertilized eggs (S419: Yes), the control flow is finished. In a case where the analysis control unit 221 determines not to finish image analysis of the fertilized eggs (S419: No), the processing from step S401 to step S419 is repeated again.

The processing to be performed by the information processing apparatus 20 in the first specific example has been described above. In the first specific example, image analysis by the respective functional units of the analyzing unit 200 is stopped for the fertilized eggs which are determined as the fertilized eggs which are poorly grown and which have poor quality. Therefore, in the first specific example, cost for image analysis by the analyzing unit 200 is reduced compared to a case where the respective functional units of the analyzing unit 200 implements image analysis for the fertilized eggs which are determined as the fertilized eggs which are poorly grown and which have poor quality. In other words, cost for image analysis by the information processing apparatus 20 is reduced. Further, in the first specific example, the imaging apparatus 10 continuously generates images of also the fertilized eggs which are determined as the fertilized eggs which are poorly grown and which have poor quality. Therefore, in a case where quality of the fertilized egg which has been determined as the fertilized egg which is poorly grown and which has poor quality is improved, the analyzing unit 200 can restart image analysis for the fertilized egg.

### <2.2.2. Second specific example>

Processing to be performed by the information processing apparatus 20 in a second specific example will be described next. In the second specific example, in step S413, the determining unit 210 determines quality of the fertilized eggs on the basis of motion amounts of the whole fertilized eggs which are one of the motion feature amounts. More specifically, the determining unit 210 determines the fertilized egg whose motion amount of the whole fertilized egg is small as the fertilized egg with poor quality. Further, in the second specific example, in step S415, the analysis control unit 221 determines to stop image analysis by the respective functional units of the analyzing unit 200 for the fertilized eggs whose quality is determined as poor. The processing to be performed by the information processing apparatus 20 in the second specific example will be described below using the flowchart in FIG. 4.

The processing from step S401 to step S411 is performed in a similar manner to that in the first specific example.

The determining unit 210 then determines quality of the respective fertilized eggs on the basis of the motion amount of the whole fertilized eggs which are one of the motion feature amounts calculated by the motion analyzing unit 203 (step S413).

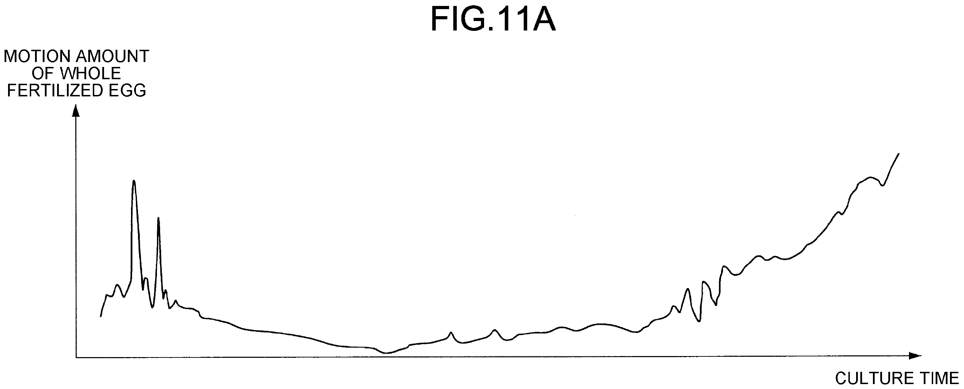

Here, a difference in temporal change of the motion amount of the whole fertilized egg between a fertilized egg with good quality and a fertilized egg with poor quality will be described. FIG. 11A is a graph illustrating an example of relationship between the motion amount of the whole fertilized egg with good quality and the culture time. As illustrated in FIG. 11A, the motion amount of the whole fertilized egg with good quality fluctuates regardless of the culture time.

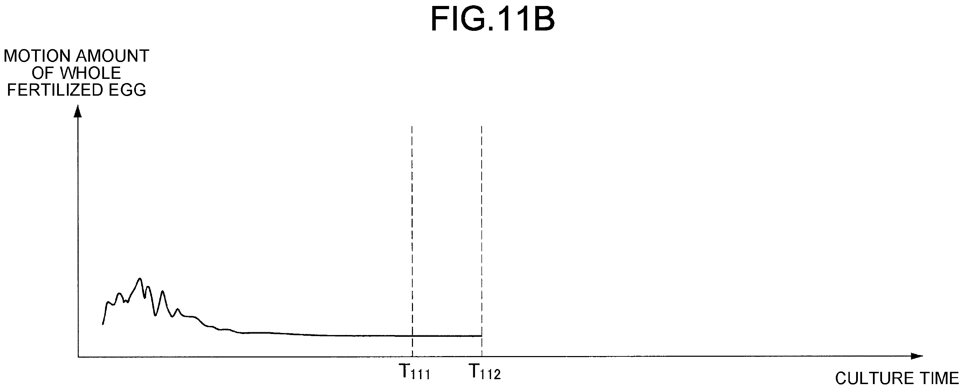

FIG. 11B is a graph illustrating an example of a result obtained by an information processing apparatus 20 in a second specific example calculating an amount of motion of the whole fertilized egg with poor quality. The motion amount is not plotted after culture time T₁₁₂ because image analysis by the analyzing unit 200 is stopped after the culture time T₁₁₂ and calculation of the motion amount of the whole fertilized egg which is one of the motion feature amounts is stopped. There is a case where the motion amount of the whole fertilized egg with poor quality becomes small in a predetermined period. For example, in the example in FIG. 11B, the motion amount of the whole fertilized egg is small from culture time T₁₁₁ to the culture time T₁₁₂, and the motion amount of the whole fertilized egg is small.

In the second specific example, in a case where the motion amount of the whole fertilized egg is small in a predetermined period, the determining unit 210 determines the fertilized egg has poor quality. In the example illustrated in FIG. 11B, the motion amount of the whole fertilized egg is small from the culture time T₁₁₁ to the culture time T₁₁₂. The determining unit 210 determines the fertilized egg which exhibits such temporal change of the motion amount of the whole fertilized egg as the fertilized egg with poor quality at the culture time T₁₁₂. In this event, the determining unit 210 transmits information of the fertilized eggs determined as the fertilized eggs with the poor quality to the control unit 220. Further, the determining unit 210 records the information of the fertilized eggs determined as the fertilized eggs with poor quality in the storage unit 230.

The analysis control unit 221 then determines the implementation conditions of image analysis to be implemented by the analyzing unit 200 on the basis of the quality of the fertilized eggs determined by the determining unit 210 (step S415). Here, description will be provided while attention is focused on a second fertilized egg which is determined as a fertilized egg with poor quality at the culture time T₁₁₂ as described above. In the second specific example, the analysis control unit 221 determines to stop image analysis by the respective functional units of the analyzing unit 200 for the fertilized egg determined as the fertilized egg which has poor quality. Note that the analysis control unit 221 can determine to continue image analysis by the respective functions of the analyzing unit 200 for the fertilized eggs which are not determined as the fertilized eggs with poor quality. Here, the determining unit 210 determines the second fertilized egg as the fertilized egg which has poor quality at the culture time T₁₁₂. Thus, the analysis control unit 221 determines to stop image analysis by the respective functional units of the analyzing unit 200 for the second fertilized egg. Calculation of the motion amounts of the whole fertilized eggs of the second fertilized egg is thereby stopped after the culture time T₁₁₂ as illustrated in FIG. 11B.

Then, in step S417, processing similar to that in the first specific example is performed. Note that, in the second specific example, information indicating that image analysis of the second fertilized egg by the respective functional units of the analyzing unit 200 is stopped may be displayed at the display apparatus 30. For example, a section corresponding to the first fertilized egg can be displayed with gray at the display apparatus 30 as illustrated in the example in FIG. 9. A user such as an embryologist can know from this display that the second fertilized egg has poor quality.

Next, in step S419, a similar processing as the first specific example is to be performed.

The processing to be performed by the information processing apparatus 20 in the second specific example has been described above. In the second specific example, image analysis by the respective functional units of the analyzing unit 200 is stopped for the fertilized eggs which are determined as the fertilized eggs which are poorly grown and which have poor quality. Therefore, in the second specific example, cost for image analysis is reduced compared to a case where the analyzing unit 200 implements image analysis for the fertilized eggs which are determined as the fertilized eggs which have poor quality. In other words, cost for image analysis by the information processing apparatus 20 is reduced. Further, in the second specific example, the imaging apparatus 10 continuously generates images of also the fertilized eggs which are determined as the fertilized eggs which have poor quality. Therefore, in a case where quality of the fertilized egg which has been determined as the fertilized egg which has poor quality is improved, the analyzing unit 200 can restart image analysis for the fertilized egg.

### <2.2.3. Third specific example>

Next, processing to be performed by the information processing apparatus 20 in the third specific example will be described. In the third specific example, in step S413, the determining unit 210 determines quality of the fertilized eggs on the basis of growth stages which are one of the morphological feature amounts. More specifically, the determining unit 210 determines a fertilized egg whose growth stage does not change for a predetermined period as the fertilized egg with poor quality. Further, in the third specific example, in step S415, the analysis control unit 221 determines to stop image analysis by the respective functional units of the analyzing unit 200 for the fertilized eggs whose quality is determined as poor. The processing to be performed by the information processing apparatus 20 in the third specific example will be described below using the flowchart in FIG. 4.

The processing from step S401 to step S411 is performed in a similar manner to that in the first specific example.

The determining unit 210 then determines quality of the fertilized eggs on the basis of the growth stages of the fertilized eggs which are one of the recognition feature amounts calculated by the morphological analyzing unit 204 (step S413).

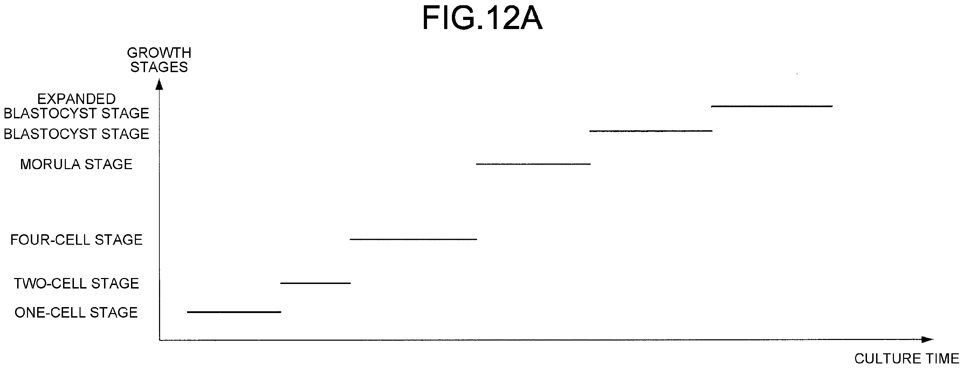

Here, a difference in temporal change of the growth stage between a fertilized egg with good quality and a fertilized egg with poor quality will be described. FIG. 12A is a graph illustrating an example of relationship between the growth stage of the fertilized egg with good quality and the culture time. As illustrated in FIG. 12A, the growth stage of the fertilized egg with good quality changes from a one-cell stage, to a two-cell stage, a four-cell stage, a morula stage, a blastocyst stage, and an expanded blastocyst stage as the culture time elapses.

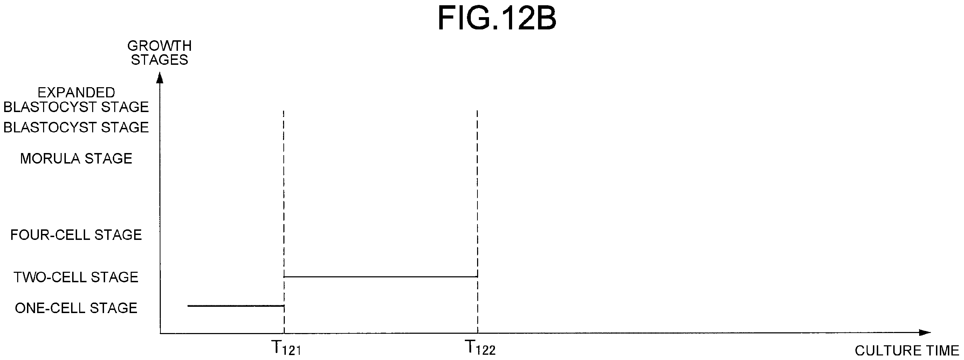

FIG. 12B is a graph illustrating an example of a result obtained by an information processing apparatus 20 in a third specific example calculating growth stages of a fertilized egg with poor quality. The growth stage is not plotted after culture time T₁₂₂ because image analysis by the analyzing unit 200 is stopped after the culture time T₁₂₂, and thus, calculation of the growth stage which is one of the morphological feature amounts is stopped. There is a case where the growth stage of the fertilized egg with poor quality does not change for a predetermined period. For example, in the example in FIG. 12B, the growth stage remains to be a two-cell stage from culture time T₁₂₁ to the culture time T₁₂₂.

In the third specific example, the determining unit 210 determines the fertilized eggs whose growth stage does not change for a predetermined period as the fertilized eggs which have poor quality. In the example illustrated in FIG. 12B, growth stage of the fertilized egg does not change for a period from the culture time T₁₂₁ to the culture time T₁₂₂. The determining unit 210 determines the fertilized eggs which exhibit such temporal change of growth stage as the fertilized eggs which have poor quality at the culture time T₁₂₂. In this event, the determining unit 210 transmits information of the fertilized eggs determined as the fertilized eggs with the poor quality to the control unit 220. Further, the determining unit 210 records the information of the fertilized eggs determined as the fertilized eggs with poor quality in the storage unit 230.

The analysis control unit 221 then determines the implementation conditions of image analysis to be implemented by the analyzing unit 200 on the basis of the quality of the fertilized eggs determined by the determining unit 210 (step S415). Here, description will be provided while attention is focused on a third fertilized egg which is determined as a fertilized egg with poor quality at the culture time T₁₂₂ as described above. In the third specific example, the analysis control unit 221 determines to stop image analysis by the respective functional units of the analyzing unit 200 for the fertilized egg determined as the fertilized egg which has poor quality. Note that the analysis control unit 221 can determine to continue image analysis by the respective functions of the analyzing unit 200 for the fertilized eggs which are not determined as the fertilized eggs with poor quality. Here, the determining unit 210 determines the third fertilized egg as the fertilized egg which has poor quality at the culture time T₁₂₂. Thus, the analysis control unit 221 determines to stop image analysis by the respective functional units of the analyzing unit 200 for the third fertilized egg. Image analysis of the third fertilized egg is thereby stopped after the culture time T₁₂₂ as illustrated in FIG. 12B.

Then, in step S417, processing similar to that in the first specific example is performed. Note that, in the third specific example, information indicating that image analysis of the third fertilized egg is stopped may be displayed at the display apparatus 30. For example, a section corresponding to the third fertilized egg can be displayed with gray at the display apparatus 30 as illustrated in the example in FIG. 9. A user such as an embryologist can know from this display that the third fertilized egg has poor quality.

Next, in step S419, a similar processing as the first specific example is to be performed.

The processing to be performed by the information processing apparatus 20 in the third specific example has been described above. In the third specific example, image analysis by the respective functional units of the analyzing unit 200 is stopped for the fertilized eggs which are determined as the fertilized eggs which are poorly grown and which have poor quality. Therefore, in the third specific example, cost for image analysis is reduced compared to a case where the analyzing unit 200 implements image analysis for the fertilized eggs which are determined as the fertilized eggs which have poor quality. In other words, cost for image analysis by the information processing apparatus 20 is reduced. Further, in the third specific example, the imaging apparatus 10 continuously generates images of also the fertilized eggs which are determined as the fertilized eggs which have poor quality. Therefore, in a case where quality of the fertilized egg which has been determined as the fertilized egg which has poor quality is improved, the analyzing unit 200 can restart image analysis for the fertilized egg.

### <2.2.4. Fourth specific example>

In a fourth specific example, the analyzing unit 200 calculates four feature amounts of each fertilized egg of a feature amount A, a feature amount B, a feature amount C and a feature amount D of the fertilized egg. These feature amounts of the fertilized egg may be any of the recognition feature amount, the motion feature amount, the morphological feature amount or the numerical feature amount, or may be combination of these. Further, in the fourth specific example, in step S415, the analysis control unit 221 determines to stop calculation of these feature amounts of the fertilized egg in a stepwise manner for the fertilized egg which is determined as the fertilized egg with poor quality. The processing to be performed by the information processing apparatus 20 in the fourth specific example will be described below using the flowchart in FIG. 4.

The processing from step S401 to step S411 is performed in a similar manner to that in the first specific example.

The determining unit 210 then determines quality of the fertilized egg on the basis of the calculated feature amounts of the fertilized egg (step S413). In the fourth specific example, the feature amounts of the fertilized egg to be used by the determining unit 210 to determine quality of the fertilized egg include at least one of the feature amount A, the feature amount B, the feature amount C or the feature amount D of the fertilized egg. In the fourth specific example, the determining unit 210 determines that quality of the fourth fertilized egg is poor at culture time T₁₃₁.

The analysis control unit 221 then determines the implementation conditions of image analysis to be implemented by the analyzing unit 200 on the basis of the quality of the fertilized egg determined by the determining unit 210 (step S415). Here, description will be provided while attention is focused on the fourth fertilized egg whose quality is determined as poor by the determining unit 210 at the culture time T₁₃₁. In the fourth specific example, the analysis control unit 221 first determines to stop calculation of the feature amount A of the fourth fertilized egg by the analyzing unit 200 after the culture time T₁₃₁. Further, the analysis control unit 221 determines to stop calculation of the feature amount B and the feature amount C of the fourth fertilized egg in addition to the feature amount A at and after culture time T₁₃₂ after time further elapses from the culture time T₁₃₁. In this manner, in the fourth specific example, the analysis control unit 221 determines to stop calculation of the feature amounts of the fertilized egg by the analyzing unit 200 in a stepwise manner.

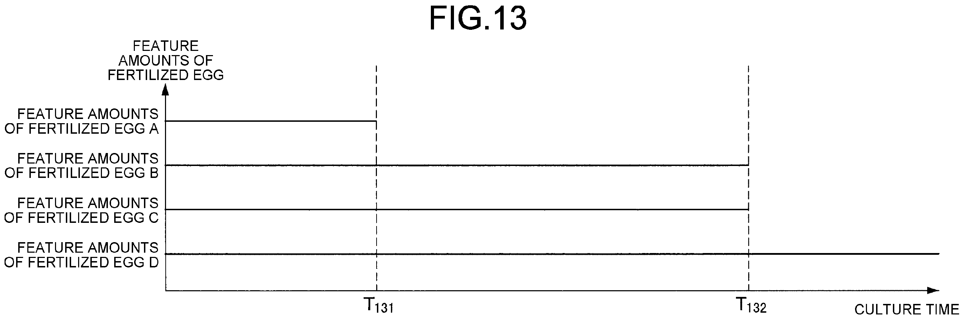

FIG. 13 is a graph for explaining an aspect where an information processing apparatus 20 in a fourth specific example stops calculation of feature amounts of fertilized eggs of fertilized eggs in a stepwise manner. FIG. 13 indicates that the respective feature amounts of the fertilized egg are calculated by the analyzing unit 200 while the feature amount A of the fertilized egg, the feature amount B of the fertilized egg, the feature amount C of the fertilized egg and the feature amount D of the fertilized egg are plotted at the culture time. In the fourth example, the analysis control unit 221 determines to stop calculation of the feature amounts of the fertilized egg in a stepwise manner. Calculation of the feature amounts of the fertilized egg to be performed by the analyzing unit 200 is thereby stopped in a stepwise manner. Specifically, the analyzing unit 200 does not calculate the feature amount A of the fourth fertilized egg at and after the culture time T₁₃₁. Further, the analyzing unit 200 stops calculation of the feature amount B and the feature amount C of the fertilized egg in addition to the feature amount A of the fertilized egg for the fourth fertilized egg at and after culture time T₁₃₂. Note that the feature amount D of the fertilized egg is continuously calculated also for the fourth fertilized egg at and after the culture time T₁₃₂. In this manner, in the fourth specific example, calculation of a plurality of feature amounts of the fertilized egg by the analyzing unit 200 is stopped in a stepwise manner.

Then, in step S417, information indicating an implementation status of image analysis is displayed at the display apparatus 30 through processing similar to that in the first specific example. Note that, in the fourth specific example, the display control unit 222 may control the communication control unit 223 to display information indicating that the feature amount A is not calculated by the analyzing unit 200 for the fourth fertilized egg at and after the culture time T₁₃₁, at the display apparatus 30. Further, the display control unit 222 may control the communication control unit 223 to display information indicating that calculation of the feature amount B and the feature amount C of the fertilized egg in addition to the feature amount A of the fertilized egg is stopped for the fourth fertilized egg at and after the culture time T₁₃₂, at the display apparatus 30.

Then, the processing in step S419 is performed in a similar manner to that in the first specific example.

The processing flow to be performed by the information processing apparatus 20 in the fourth specific example has been described above. As described above, as a result of calculation of the feature amounts of the fertilized egg which is determined as the fertilized egg with poor quality being stopped in a stepwise manner, cost required for image analysis of the fertilized eggs is reduced in a stepwise manner. Consequently, cost required for image analysis is reduced as the culture time elapses. Further, the analysis control unit 221 can determine to restart calculation for the feature amount of the fertilized egg for which calculation has been stopped depending on temporal change of the feature amounts of the fertilized egg which are continuously calculated. Calculation of the feature amount of the fertilized egg which has been stopped once is thereby restarted.

### <2.3.5. Fifth specific example>

In a fifth specific example, in step S415, the analysis control unit 221 determines to reduce the number of analysis methods to be used in calculation of the feature amounts of the fertilized egg which is determined as the fertilized egg with poor quality, in a stepwise manner. Processing to be performed by the information processing apparatus 20 in the fifth specific example will be described below using the flowchart in FIG. 4.

Step S401 to step S411 are performed in a similar manner to that in the first specific example.

The determining unit 210 then determines quality of each fertilized egg on the basis of the feature amounts calculated by the analyzing unit 200 (step S413). In the fifth specific example, the feature amounts to be used in determination of quality of the fertilized egg may be any of the recognition feature amount, the motion feature amount, the morphological feature amount or the numerical feature amount, or combination of these. In the fifth embodiment, the determining unit 210 determines that quality of a fifth fertilized egg is poor at culture time T₁₄₁.

The analysis control unit 221 then determines implementation conditions of image analysis to be implemented by the analyzing unit 200 on the basis of the determination result of the determining unit 210 (step S415). Here, description will be provided while attention is focused on the fifth fertilized egg which is determined as the fertilized egg with poor quality at the culture time T₁₄₁. In the fifth specific example, the analysis control unit 221 determines to reduce the number of analysis methods to be used in image analysis in a stepwise manner for the fertilized egg which is determined as the fertilized egg with poor quality. Here, the determining unit 210 determines the fifth fertilized egg as the fertilized egg with poor quality at the culture time T₁₄₁. The analysis control unit 221 therefore determines to reduce the number of analysis methods to be used in calculation of the feature amounts of the fifth fertilized egg in a stepwise manner on the basis of the determination result.

First, the analysis control unit 221 determines to stop motion analysis which is one of the analysis methods of image analysis, for the fifth fertilized egg. Further, the analysis control unit 221 determines to stop morphological analysis which is one of the analysis methods to be used in image analysis, for the fifth fertilized egg at and after culture time T₁₄₂ after time further elapses from the culture time T₁₄₁. In this manner, in the fifth specific example, the analysis control unit 221 determines to stop the analysis methods to be used in image analysis in a stepwise manner for the fertilized egg determined as the fertilized egg with poor quality.

Note that the order of various kinds of analysis methods being stopped may be determined in advance. Further, the order of various kinds of analysis methods being stopped may be determined in accordance with temporal change of the feature amounts of the fertilized egg.

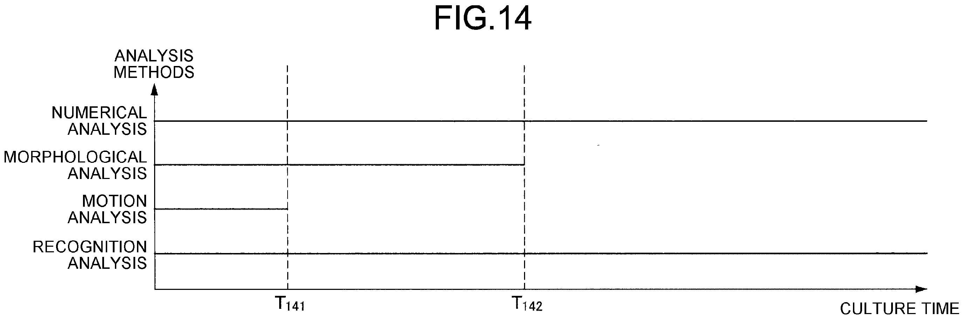

FIG. 14 is a graph for explaining an aspect where an information processing apparatus 20 in a fifth specific example stops analysis methods to be used in image analysis of fertilized eggs in a stepwise manner. As illustrated in FIG. 14, in the fifth specific example, the analysis methods to be used in image analysis for the fertilized egg determined as the fertilized egg with poor quality are stopped in a stepwise manner. In the fifth specific example, the analyzing unit 200 stops motion analysis for the fifth fertilized egg at and after the culture time T₁₄₁. Further, the analyzing unit 200 stops morphological analysis in addition to the motion analysis for the fifth fertilized egg at and after the culture time T₁₄₂. In this manner, in the fifth specific example, the analysis methods to be used in image analysis for the fertilized egg are reduced in a stepwise manner.

Then, in step S417, information indicating an implementation status of image analysis is displayed at the display apparatus 30 by performing processing similar to that in the first specific example. Note that, in the fifth specific example, the display control unit 222 may control the communication control unit 223 so as to display information indicating that motion analysis is not performed for the fifth fertilized egg at and after the culture time T₁₄₁, at the display apparatus 30. Further, the display control unit 222 may control the communication control unit 223 so as to display information indicating that morphological analysis in addition to the motion analysis is stopped for the fifth fertilized egg at and after the culture time T₁₄₂, at the display apparatus 30.

Then, the processing in step S419 is performed in a similar manner to that in the first specific example.

The processing flow to be performed by the information processing apparatus 20 in the fifth specific example has been described above. As described above, as a result of the analysis methods to be used in image analysis being stopped for the fertilized egg determined as the fertilized egg with poor quality in a stepwise manner, cost required for image analysis of the fertilized eggs is reduced in a stepwise manner. Consequently, cost required for image analysis is reduced as the culture time elapses. Further, the analysis control unit 221 can determine to restart the analysis method which has been stopped depending on temporal change of the feature amounts of the fertilized egg calculated using the analysis methods which are not stopped. The analysis method which has been stopped once can be thereby restarted.

Note that the analysis control unit 221 may reduce the number of analysis methods to be used in image analysis in a stepwise manner as in the specific example 5 instead of stopping image analysis for the fertilized egg which is determined as the fertilized egg with poor quality due to small temporal change of the area of the fertilized egg which is one of the recognition feature amounts of the fertilized egg as in the specific example 1. Further, the analysis control unit 221 may reduce the number of analysis methods to be used in image analysis in a stepwise manner as in the specific example 5 instead of stopping image analysis for the fertilized egg which is determined as the fertilized egg with poor quality due to a small motion amount of the whole fertilized egg which is one of the motion feature amounts of the fertilized egg for a predetermined period as in the specific example 2. Still further, the analysis control unit 221 may reduce the number of analysis methods to be used in image analysis in a stepwise manner as in the specific example 5 instead of stopping image analysis for the fertilized egg which is determined as the fertilized egg with poor quality due to no change in the growth state which is one of the morphological feature amounts as in the specific example 3. Alternatively, the analysis control unit 221 may stop image analysis after reducing the number of analysis methods to be used in image analysis in a stepwise manner for the fertilized egg which is determined as the fertilized egg with poor quality as in the specific examples 1 to 3.

Further, the analysis control unit 221 may reduce the number of analysis methods to be used in image analysis in a stepwise manner after reducing the number of feature amounts of the fertilized egg to be calculated in a stepwise manner as in the specific example 4 for the fertilized egg which is determined as the fertilized egg with poor quality. Alternatively, the analysis control unit 221 may reduce the number of feature amounts of the fertilized egg to be calculated in a stepwise manner as in the specific example 4 after (or at the same time as) reducing the number of analysis methods to be used in image analysis in a stepwise manner for the fertilized egg which is determined as the fertilized egg with poor quality.

### <2.2.6. Sixth specific example>

In a sixth specific example, in step S415, the analysis control unit 221 performs control to interrupt image analysis so that a period during which the analyzing unit 200 does not implement image analysis becomes constant regardless of elapsed time for the fertilized egg which is determined as the fertilized egg with poor quality. In other words, the analysis control unit 221 performs control to interrupt image analysis for the fertilized egg which is determined as the fertilized egg with poor quality while setting a period during which the analyzing unit 200 does not implement image analysis as a predetermined culture time interval. Processing to be performed by the information processing apparatus 20 in the sixth specific example will be described below using the flowchart in FIG. 4.

The processing from steps S401 to S411 is performed a similar processing as in the first specific example.

The determining unit 210 then determines quality of each fertilized egg on the basis of the feature amounts analyzed by the analyzing unit 200 (step S413). In the sixth specific example, the feature amounts to be used in determination of quality of the fertilized egg may be any of the recognition feature amount, the motion feature amount, the morphological feature amount or the numerical feature amount, or combination of these. In the sixth specific example, the determining unit 210 determines that quality of a sixth fertilized egg is poor at culture time T₁₅₁.

The analysis control unit 221 then determines implementation conditions of image analysis by the analyzing unit 200 on the basis of the determination result of the determining unit 210 (step S415). Here, description will be provided while attention is focused on a sixth fertilized egg which is determined by the determining unit 210 as the fertilized egg with poor quality at culture time T₁₅₁. In the sixth specific example, the analysis control unit 221 determines to interrupt image analysis at predetermined culture time intervals Δt for the fertilized egg which is determined as the fertilized egg with poor quality. In the sixth specific example, the analysis control unit 221 determines to interrupt image analysis at and after the culture time T₁₅₁ at predetermined culture time intervals Δt for the sixth fertilized egg which is determined by the determining unit 210 as the sixth fertilized egg with poor quality at the culture time T₁₅₁. Note that the culture time interval Δt is a fixed time interval regardless of the culture time. Note that a culture time interval at which image analysis is implemented is also a fixed time interval regardless of the culture time.

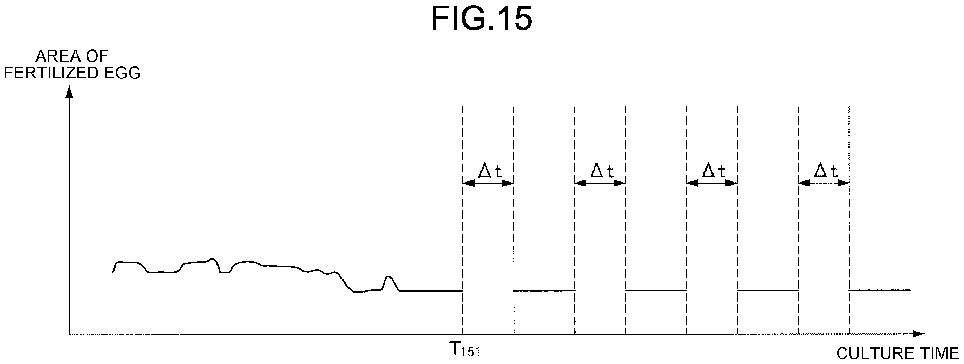

FIG. 15 is a graph for explaining an aspect where an information processing apparatus 20 in a sixth specific example interrupts image analysis of fertilized eggs with poor quality at predetermined culture time intervals Δt. A graph in FIG. 15 indicates an area of the fertilized egg on a vertical axis as one example of the feature amount of the fertilized egg for which image analysis is interrupted. As illustrated in FIG. 15, calculation of an area of the sixth fertilized egg by the recognition analyzing unit 202 is interrupted at culture time intervals Δt at and after the culture time T₁₅₁ at which the determining unit 210 determines that the sixth fertilized egg has poor quality.

Then, in step S417, information indicating an implementation status of image analysis is displayed at the display apparatus 30 by performing processing similar to that in the first specific example. Note that the display control unit 222 may control the communication control unit 223 so as to display information indicating that image analysis is interrupted for the sixth fertilized egg at and after the culture time T₁₅₁, at the display apparatus 30.

Next, in step S419, a similar processing as the first specific example is to be performed.

The processing flow to be performed by the information processing apparatus 20 in the sixth specific example has been described above. In this manner, as a result of image analysis of the fertilized egg being interrupted at predetermined culture time intervals Δt, cost required for image analysis of the fertilized egg is reduced. Further, there is also a period during which image analysis is implemented while image analysis is interrupted. The analysis control unit 221 can therefore perform control the analyzing unit 200 to continuously implement image analysis on the basis of the feature amounts of the fertilized egg calculated while the image analysis is implemented. Consequently, it becomes possible to continuously implement image analysis again also for the fertilized egg for which image analysis has been interrupted once.

Note that the analysis control unit 221 may interrupt calculation of part of the feature amounts of the fertilized egg calculated for the fertilized egg which is determined as the fertilized egg with poor quality at predetermined culture time intervals. Further, the analysis control unit 221 may interrupt part of the analysis methods to be used in image analysis for the fertilized egg which is determined as the fertilized egg with poor quality at predetermined culture time intervals.

Note that the analysis control unit 221 may interrupt image analysis at predetermined culture time intervals in a stepwise manner as in the specific example 6 instead of stopping image analysis for the fertilized egg which is determined as the fertilized egg with poor quality due to small temporal change of the area of the fertilized egg which is one of the recognition feature amounts of the fertilized egg as in the specific example 1. Further, the analysis control unit 221 may interrupt image analysis at predetermined culture time intervals in a stepwise manner as in the specific example 6 instead of stopping image analysis for the fertilized egg which is determined as the fertilized egg with poor quality due to a small motion amount of the whole fertilized egg which is one of the motion feature amounts of the fertilized egg for a predetermined period as in the specific example 2. Still further, the analysis control unit 221 may interrupt image analysis at predetermined culture time intervals in a stepwise manner as in the specific example 6 instead of stopping image analysis for the fertilized egg which is determined as the fertilized egg with poor quality due to no change in the growth state which is one of the morphological feature amounts as in the specific example 3. Alternatively, the analysis control unit 221 may stop image analysis after interrupting image analysis at predetermined culture time intervals in a stepwise manner for the fertilized egg which is determined as the fertilized egg with poor quality as in the specific examples 1 to 3.

Further, the analysis control unit 221 may interrupt image analysis at predetermined culture time intervals in a stepwise manner after reducing the number of feature amounts of the fertilized egg to be calculated in a stepwise manner as in the specific example 4 for the fertilized egg which is determined as the fertilized egg with poor quality. Alternatively, the analysis control unit 221 may reduce the number of feature amounts of the fertilized egg to be calculated in a stepwise manner as in the specific example 4 after (or at the same time as) interrupting image analysis at predetermined culture time intervals in a stepwise manner for the fertilized egg which is determined as the fertilized egg with poor quality.

Further, the analysis control unit 221 may reduce the number of analysis methods to be used in image analysis as in the specific example 5 after interrupting image analysis at predetermined time intervals for the fertilized egg which is determined as the fertilized egg with poor quality. Alternatively, the analysis control unit 221 may interrupt image analysis at predetermined culture time intervals after (or at the same time as) the number of analysis methods to be used in image analysis is reduced as in the specific example 5.

### <2.2.7. Seventh specific example>

In a seventh specific example, in step S415, the analysis control unit 221 determines to interrupt image analysis so that a period during which image analysis is not implemented becomes longer as the culture time elapses for the fertilized egg which is determined as the fertilized egg with poor quality. Processing to be performed by the information processing apparatus 20 in the seventh specific example will be described below using the flowchart in FIG. 4.

First, in from step S401 to step S411, a processing similar to that in the first specific example is performed.

The determining unit 210 then determines quality of each fertilized egg on the basis of the feature amounts calculated by the analyzing unit 200 (step S413). In the seventh specific example, the feature amounts to be used in determination of quality of the fertilized egg may be any of the recognition feature amount of fertilized eggs, the motion feature amount, the morphological feature amount or the numerical feature amount, or combination of these. In the seventh specific example, the determining unit 210 determines that quality of a seventh fertilized egg is poor at culture time T₁₆₁.

The analysis control unit 221 then determines implementation conditions of image analysis to be implemented by the analyzing unit 200 on the basis of the determination result of the determining unit 210 (step S415). Here, description will be provided while attention is focused on a seventh fertilized egg which is determined as the fertilized egg with poor quality at culture time T₁₆₁. In the seventh specific example, the analysis control unit 221 determines to interrupt image analysis so that a period during which image analysis is not implemented becomes longer as the culture time elapses for the fertilized egg which is determined as the fertilized egg with poor quality. In the seventh specific example, the analysis control unit 221 determines to interrupt image analysis so that a period during which image analysis is not implemented becomes longer as the culture time elapses for the seventh fertilized egg which is determined by the determining unit 210 as the seventh fertilized egg with poor quality at the culture time T₁₆₁.

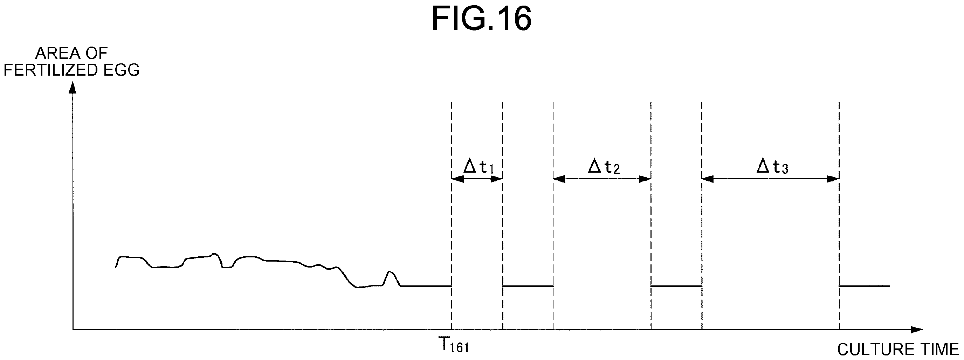

FIG. 16 is a graph for explaining an aspect where an information processing apparatus 20 in a seventh specific example interrupts image analysis of fertilized eggs with poor quality so that a period during which image analysis is not implemented becomes longer as time elapses.

As illustrated in FIG. 16, calculation of an area of the seventh fertilized egg by the recognition analyzing unit 202 is interrupted at and after the culture time T₁₆₁. As illustrated in FIG. 16, the culture time intervals Δt₁, Δt₂ and Δt₃ during which calculation of the area of the fertilized egg is stopped becomes longer as the culture time elapses. In this manner, in the seventh specific example, image analysis is interrupted so that a time interval during which the analyzing unit 200 does not implement image analysis becomes longer as the culture time elapses.

Then, in step S417, information indicating an implementation status of image analysis is displayed at the display apparatus 30 by performing processing similar to that in the first specific example. Note that the display control unit 222 may control the communication control unit 223 so as to display information indicating that image analysis is interrupted for the seventh fertilized egg at and after the culture time T₁₆₁, at the display apparatus 30.

Next, in step S419, a processing is to be performed similarly to the first specific example.

As described above, in the seventh specific example, image analysis is interrupted so that a time interval during which image analysis of the fertilized egg is not implemented becomes longer as the culture time elapses. Cost required for image analysis by the information processing apparatus 20 is thereby reduced. In the seventh specific example, a period during which image analysis is not implemented becomes longer as the culture time elapses, so that cost for image analysis is reduced as the culture time elapses. Further, there is also a period during which image analysis is implemented while image analysis is interrupted. The analysis control unit 221 can therefore perform control the analyzing unit 200 to continuously implement image analysis on the basis of the feature amounts of the fertilized egg calculated while the image analysis is implemented. Consequently, it becomes possible to continuously implement image analysis again also for the fertilized egg for which image analysis has been interrupted once.

Note that the analysis control unit 221 may interrupt calculation of part of the feature amounts of the fertilized egg to be calculated so that a period during which calculation is not performed becomes longer as the culture time elapses for the fertilized egg which is determined as the fertilized egg with poor quality. Further, the analysis control unit 221 may interrupt part of the analysis methods to be used in image analysis so that a period during which the analysis method is not performed becomes longer as the culture time elapses for the fertilized egg which is determined as the fertilized egg with poor quality.

Note that the analysis control unit 221 may interrupt image analysis so that a period during which image analysis is not implemented becomes longer as the culture time elapses in a stepwise manner as in the specific example 7 instead of stopping image analysis for the fertilized egg which is determined as the fertilized egg with poor quality due to small temporal change of the area of the fertilized egg which is one of the recognition feature amounts of the fertilized egg as in the specific example 1. Further, the analysis control unit 221 may interrupt image analysis so that a period during which image analysis is not implemented becomes longer as the culture time elapses in a stepwise manner as in the specific example 7 instead of stopping image analysis for the fertilized egg which is determined as the fertilized egg with poor quality due to a small motion amount of the whole fertilized egg which is one of the motion feature amounts of the fertilized egg for a predetermined period as in the specific example 2. Still further, the analysis control unit 221 may interrupt image analysis so that a period during which image analysis is not implemented becomes longer as the culture time elapses in a stepwise manner as in the specific example 7 instead of stopping image analysis for the fertilized egg which is determined as the fertilized egg with poor quality due to no change in the growth state which is one of the morphological feature amounts as in the specific example 3. Alternatively, the analysis control unit 221 may stop image analysis after interrupting image analysis so that a period during which image analysis is not implemented becomes longer as the culture time elapses in a stepwise manner for the fertilized egg which is determined as the fertilized egg with poor quality as in the specific examples 1 to 3.

Further, the analysis control unit 221 may interrupt image analysis so that a period during which image analysis is not implemented becomes longer as the culture time elapses in a stepwise manner after reducing the number of feature amounts of the fertilized egg to be calculated in a stepwise manner as in the specific example 4 for the fertilized egg which is determined as the fertilized egg with poor quality. Alternatively, the analysis control unit 221 may reduce the number of feature amounts of the fertilized egg to be calculated in a stepwise manner as in the specific example 4 after (or at the same time as) interrupting image analysis so that a period during which image analysis is not implemented becomes longer as the culture time elapses in a stepwise manner for the fertilized egg which is determined as the fertilized egg with poor quality.

Further, the analysis control unit 221 may reduce the number of analysis methods to be used in image analysis as in the specific example 5 after interrupting image analysis so that a period during which image analysis is not implemented becomes longer as the culture time elapses for the fertilized egg which is determined as the fertilized egg with poor quality. Alternatively, the analysis control unit 221 may interrupt image analysis so that a period during which image analysis is not implemented becomes longer as the culture time elapses after (or at the same time as) the number of analysis methods to be used in image analysis is reduced as in the specific example 5.

Further, the analysis control unit 221 may interrupt image analysis at predetermined culture time intervals as in the specific example 6 after interrupting image analysis so that a period during which image analysis is not implemented becomes longer as the culture time elapses for the fertilized egg which is determined as the fertilized egg with poor quality. Alternatively, the analysis control unit 221 may interrupt image analysis at predetermined culture time intervals as in the specific example 6 after (or at the same time as) image analysis is interrupted so that a period during which image analysis is not implemented becomes longer as the culture time elapses.

The specific examples of the processing to be performed by the information processing apparatus 20 according to an embodiment of the present disclosure have been described above. Note that determination by the determining unit 210 or control of the implementation conditions by the analysis control unit 221 performed in the specific examples 1 to 7 may be performed in combination of any two, or three or more specific examples unless logical inconsistency arises.

### <2.3. Effects>

The processing examples where the information processing apparatus 20 according to an embodiment of the present disclosure implements image analysis on the feature amounts of the fertilized eggs from captured images of a plurality of fertilized eggs have been described above. Next, effects of the information processing apparatus 20 according to an embodiment of the present disclosure will be described.

With the technology disclosed in Patent Literature 1, an image of a specimen which is in a state inappropriate for measurement is not captured. Meanwhile, in the information processing system 1 according to an embodiment of the present disclosure, the imaging apparatus 10 captures images of all fertilized eggs cultured in the culture medium M1 regardless of the calculated feature amounts of the fertilized eggs. The information processing apparatus 20 thereafter controls implementation conditions of image analysis of the fertilized eggs in accordance with the calculated feature amounts of the fertilized eggs.

Here, it is necessary to irradiate the fertilized eggs with light having a red wavelength, a near infrared wavelength, or the like, so that the imaging apparatus 10 captures images of the fertilized eggs. Typically, fertilized eggs are vulnerable to light, and there is a case where quality of fertilized eggs degrades if fertilized eggs are irradiated with light for a long period of time. Particularly, in a case where an image of a large number of fertilized eggs is captured, if whether or not it is necessary to capture an image of each specimen is determined by capturing an image of each specimen as in the technology disclosed in Patent Literature 1, it takes time to capture an image, which leads to a possibility that quality of fertilized eggs may degrade while the image is captured. Meanwhile, in the information processing system 1 according to the present embodiment, the imaging apparatus 10 only has to capture an image of all fertilized eggs once, so that it is possible to generate images including all the fertilized eggs with less times of image capture by the imaging apparatus 10 capturing an image of a large number of fertilized eggs at one time. This shortens a period during which the fertilized eggs are irradiated with light and prevents quality of the fertilized eggs from degrading.

Further, in the information processing system 1 according to an embodiment of the present disclosure, the analyzing unit 200 can analyze images including a large number of fertilized eggs cultured in the culture medium M1. It is therefore possible to implement image analysis on all the fertilized eggs through analysis with less images even if there are a large number of fertilized eggs, so that it is possible to efficiently implement image analysis.

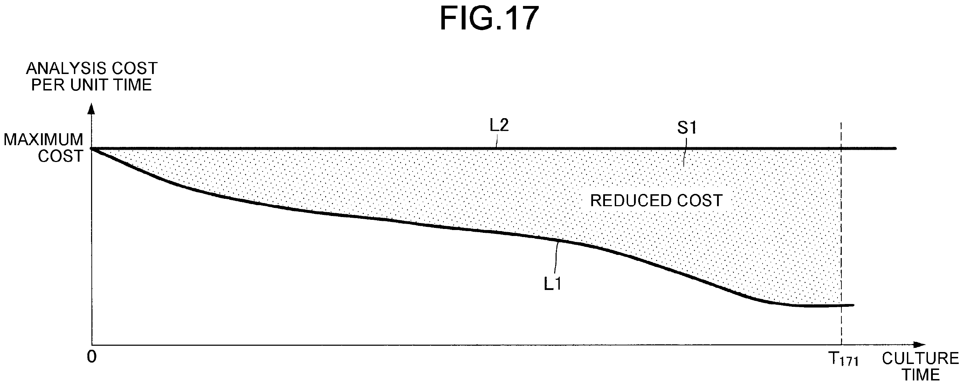

FIG. 17 is a graph for explaining reduction of cost required for image analysis by the information processing apparatus 20 according to an embodiment of the present disclosure. FIG. 17 indicates analysis cost per unit time (frame) on a vertical axis and indicates the culture time on a horizontal axis. The analysis cost is cost required for analyzing the images of the fertilized eggs. Further, maximum cost indicated on the vertical axis in FIG. 17 is analysis cost per unit time at a time point at which the information processing apparatus 20 starts all kinds of image analysis of the recognition analysis, the motion analysis, the morphological analysis and the numerical analysis. A curve L1 indicates relationship between the analysis cost per unit time by the information processing apparatus 20 according to an embodiment of the present disclosure and the culture time.

In a case where the information processing apparatus 20 does not change the number of feature amounts to be calculated through image analysis and the number of analysis methods to be used in image analysis regardless of quality of the fertilized eggs, the analysis cost per unit time is fixed at the maximum cost as indicated with a line L2.

Meanwhile, in the information processing apparatus 20 according to an embodiment of the present disclosure, the analysis control unit 221 controls implementation conditions of image analysis by the analyzing unit 200 on the basis of the feature amounts of the fertilized eggs. More specifically, the analysis control unit 221 determines to stop, interrupt, or the like, image analysis for the fertilized egg which is determined as the fertilized egg with poor quality. The analyzing unit 200 stops, interrupts, or the like, image analysis for the fertilized egg which is determined as the fertilized egg with poor quality on the basis of this determination. As a result of image analysis of the fertilized eggs being stopped, interrupted, or the like, by the information processing apparatus 20 according to an embodiment of the present disclosure, analysis time and cost of a computer to be used to implement image analysis of the fertilized eggs, cost, or the like, for storing analysis results are reduced. As a result, analysis cost per unit time is reduced.

Further, in a case where the number of fertilized eggs for which image analysis is stopped, interrupted, or the like, increases as the culture time elapses, analysis cost per unit time by the information processing apparatus 20 decreases as the culture time elapses. As an example, a case will be described where the analyzing unit 200 stops image analysis of the fertilized eggs with poor quality. In this case, image analysis is stopped for the fertilized eggs with poor quality as time elapses, and the number of fertilized eggs for which image analysis is implemented decreases. If quality of 80% of the fertilized eggs out of the entire fertilized eggs is poor, if image analysis is stopped for all the fertilized eggs with poor quality, image analysis is implemented on 20% of the fertilized eggs by excluding the fertilized eggs with poor quality from the entire fertilized eggs. As a result, cost per unit time is reduced to cost required for implementing image analysis on 20% of the entire fertilized eggs.

Further, in a case where the number of feature amounts of the fertilized eggs for which calculation is stopped increases in a stepwise manner as in the fourth specific example, analysis cost per unit time decreases every time the number of feature amounts of the fertilized eggs for which calculation is stopped increases. Still further, in a case where the analysis methods to be used in image analysis is reduced in a stepwise manner as in the fifth specific example, analysis cost per unit time decreases every time the analysis methods to be used in image analysis is reduced.

As described above, in the information processing apparatus 20 according to an embodiment of the present disclosure, analysis cost per unit time decreases as the culture time elapses. As a result, analysis cost per unit time (unit frame) decreases as the culture time elapses as indicated with the curve L1. As illustrated in FIG. 17, cost required for implementing image analysis on the fertilized eggs from when culture of the fertilized eggs is started until culture time T₁₇₁ is reduced by an amount corresponding to an area S1 enclosed with the curve L1, a line L2 and a dashed line at the culture time T₁₇₁ by the information processing apparatus 20 according to an embodiment of the present disclosure. In other words, in image analysis of fertilized eggs by the information processing apparatus 20 according to an embodiment of the present disclosure, cost required for implementing image analysis on fertilized eggs is reduced by an amount corresponding to cost corresponding to the area S1.

Further, in the information processing apparatus 20 according to an embodiment of the present disclosure, images of the fertilized eggs are continuously generated by the imaging apparatus 10 also for the fertilized eggs for which image analysis has been stopped. Consequently, the information processing apparatus 20 can restart image analysis also for the fertilized eggs for which image analysis has been stopped on the basis of the feature amounts of the fertilized eggs. For example, the information processing apparatus 20 can restart image analysis in a case where quality of the fertilized egg which has been determined as the fertilized egg with poor quality once and for which image analysis has been stopped, is determined as good thereafter. In this manner, the information processing apparatus 20 can appropriately analyze the images of the fertilized eggs, and thus can deal with the fertilized eggs which are analysis targets more appropriately.

The effects of the information processing apparatus 20 according to an embodiment of the present disclosure have been described above.

### <<3. Hardware configuration example>>

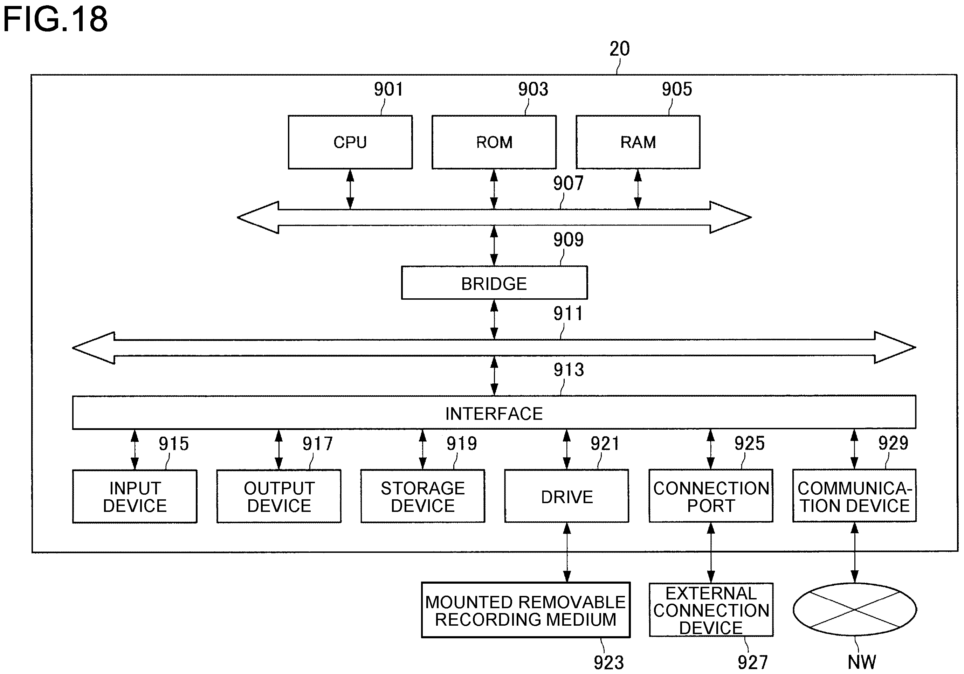

Next, with reference to FIG. 18, a hardware configuration of an information processing apparatus according to an embodiment of the present disclosure is described. FIG. 18 is a block diagram illustrating an exemplary hardware configuration of the information processing apparatus of an embodiment of the present disclosure. An illustrated information processing apparatus 20 can realize the information processing apparatus 20 in the above described embodiment.

The information processing apparatus 20 includes a CPU 901, a read only memory (ROM) 903, and a random access memory (RAM) 905. In addition, the information processing apparatus 20 may include a host bus 907, a bridge 909, an external bus 911, an interface 913, an input device 915, an output device 917, a storage device 919, a drive 921, a connection port 925, and a communication device 929. The information processing apparatus 20 may include a processing circuit such as a digital signal processor (DSP) or an application specific integrated circuit (ASIC), instead of or in addition to the CPU 901.

The CPU 901 functions as an arithmetic processing device and a control device, and controls the overall operation or a part of the operation of the information processing apparatus 20 according to various programs recorded in the ROM 903, the RAM 905, the storage device 919, or a removable recording medium 923. For example, the CPU 901 controls overall operations of respective function units included in the information processing apparatus 20 of the above-described embodiment. The ROM 903 stores programs, operation parameters, and the like used by the CPU 901. The RAM 905 transiently stores programs used when the CPU 901 is executed, and parameters that change as appropriate when executing such programs. The CPU 901, the ROM 903, and the RAM 905 are connected with each other via the host bus 907 configured from an internal bus such as a CPU bus or the like. The host bus 907 is connected to the external bus 911 such as a Peripheral Component Interconnect/Interface (PCI) bus via the bridge 909.

The input device 915 is a device operated by a user such as a mouse, a keyboard, a touchscreen, a button, a switch, and a lever. The input device 915 may be a remote control device that uses, for example, infrared radiation and another type of radio waves. Alternatively, the input device 915 may be an external connection device 927 such as a mobile phone that corresponds to an operation of the information processing apparatus 20. The input device 915 includes an input control circuit that generates input signals on the basis of information which is input by a user to output the generated input signals to the CPU 901. The user inputs various types of data and indicates a processing operation to the information processing apparatus 20 by operating the input device 915.

The output device 917 includes a device that can visually or audibly report acquired information to a user. The output device 917 may be, for example, a display apparatus such as an LCD, a PDP, and an OELD, an audio output device such as a speaker and a headphone, and a printer. The output device 917 outputs a result obtained through a process performed by the information processing apparatus 20, in the form of text or video such as an image, or sounds such as audio sounds.

The storage device 919 is a device for data storage that is an exemplary storage unit of the information processing apparatus 20. The storage device 919 includes, for example, a magnetic storage device such as a hard disk drive (HDD), a semiconductor storage device, an optical storage device, or a magneto-optical storage device. The storage device 919 stores therein the programs and various data executed by the CPU 901, and various data acquired from an outside.

The drive 921 is a reader/writer for the removable recording medium 923 such as a magnetic disk, an optical disc, a magneto-optical disk, or a semiconductor memory, and built in or externally attached to the information processing apparatus 20. The drive 921 reads out information recorded on the mounted removable recording medium 923, and outputs the information to the RAM 905. The drive 921 writes the record into the mounted removable recording medium 923.

The connection port 925 is a port used to directly connect devices to the information processing apparatus 20. The connection port 925 may be a Universal Serial Bus (USB) port, an IEEE1394 port, or a Small Computer System Interface (SCSI) port, for example. The connection port 925 may also be an RS-232C port, an optical audio terminal, a High-Definition Multimedia Interface (HDMI (registered trademark)) port, and so on. The connection of the external connection device 927 to the connection port 925 makes it possible to exchange various kinds of data between the information processing apparatus 20 and the external connection device 927.

The communication device 929 is a communication interface including, for example, a communication device for connection to a communication network NW. The communication device 929 may be, for example, a wired or wireless local area network (LAN), Bluetooth (registered trademark), or a communication card for a wireless USB (WUSB). The communication device 929 may also be, for example, a router for optical communication, a router for asymmetric digital subscriber line (ADSL), or a modem for various types of communication. For example, the communication device 929 transmits and receives signals in the Internet or transits signals to and receives signals from another communication device by using a predetermined protocol such as TCP/IP. The communication network NW to which the communication device 929 connects is a network established through wired or wireless connection. The communication network NW is, for example, the Internet, a home LAN, infrared communication, radio wave communication, or satellite communication.

Note that the CPU 901, the ROM 903 and the RAM 905 and so forth can enable the functions of the control unit 220 according to the embodiment. Meanwhile the storage device 919 can enable the function of the storage unit 230 according to the embodiment. Moreover, at least either the connection port 925 or the communication device 929 can enable the function of the communication control unit 223 according to the embodiment.

The example of the hardware configuration of the information processing apparatus 20 has been introduced.

### <<4. Conclusion>>

As described above, the favorable embodiments of the present disclosure have been described in detail with reference to the accompanying drawings, but the technical scope of the present disclosure is not limited to such examples. A person skilled in the art may find various alterations and modifications within the scope of the appended claims, and it should be understood that they will naturally come under the technical scope of the present disclosure.

For example, although the information processing system 1 is configured to be provided with the imaging apparatus 10 and information processing apparatus 20 in the above-described embodiment, the present technology is not limited thereto. For example, the imaging apparatus 10 may have the function of the information processing apparatus 20 (For example, function to analyze image, function to determine the state of the cell, and function to determine implementation conditions of image analysis). In this case, the information processing system 1 is embodied by the imaging apparatus 10. In addition, the information processing apparatus 20 may have the function of the imaging apparatus 10 (imaging function). In this case, the information processing system 1 is embodied by the information processing apparatus 20. Further, the imaging apparatus 10 may have a part of the function of the information processing apparatus 20, and the information processing apparatus 20 may have a part of the function of the imaging apparatus 10.

Also, a computer program causing hardware such as the CPU, the ROM, and the RAM included in the information processing apparatus to carry out the equivalent functions as the above-described configuration of the information processing apparatus can be generated. Also, a readable recording medium having the computer program stored therein can be provided.

Furthermore, the effects described in the present specification are merely illustrative or exemplary and are not restrictive. That is, the technology according to the present disclosure can exhibit other effects obvious to those skilled in the art from the description of the present specification in addition to or in place of the above-described effects.

Note that the following configuration also belong to the technical scope of the present disclosure.
(1) An information processing apparatus comprising:
   an analyzing unit configured to analyze a plurality of images obtained by capturing images of an observation target including a cell having ability to divide in chronological order, to calculate a feature amount representing a state of the cell; and
   a control unit configured to dynamically control an implementation condition of the analysis on a basis of the calculated feature amount.
(2) The information processing apparatus according to (1), further comprising: a determining unit configured to determine the state of the cell on a basis of the feature amount.
(3) The information processing apparatus according to (2), wherein the determining unit determines the state of the cell using a predetermined machine learning algorithm.
(4) The information processing apparatus according to (2) or (3), wherein the control unit dynamically controls the implementation condition of the analysis on a basis of the state of the cell determined from the feature amount.
(5) The information processing apparatus according to any one of (1) to (3), wherein the control unit dynamically controls the implementation condition of the analysis on a basis of temporal change of the feature amount.
(6) The information processing apparatus according to any one of (1) to (5), wherein the control unit performs control to interrupt implementation timing of the analysis.
(7) The information processing apparatus according to (6), wherein the control unit performs control so that a period during which the analysis is not implemented becomes fixed regardless of elapsed time.
(8) The information processing apparatus according to (6), wherein the control unit performs control so that a period during which the analysis is not implemented becomes longer as time elapses.
(9) The information processing apparatus according to any one of (1) to (5), wherein the control unit performs control to stop implementation of the analysis.
(10) The information processing apparatus according to any one of (1) to (5), wherein the control unit performs control to reduce a number of the feature amounts to be calculated as time elapses.
(11) The information processing apparatus according to any one of (1) to (5), wherein the control unit performs control to reduce a number of predetermined analysis methods to be used in the analysis as time elapses.
(12) The information processing apparatus according to any one of (1) to (11), wherein the feature amount representing the state of the cell includes at least one of a recognition feature amount which is information regarding a shape of the cell, a motion feature amount which is information characterizing motion of the cell, a morphological feature amount which is information characterizing morphology of the cell, or a numerical feature amount which is a numerical value representing a state of a fertilized egg.
(13) The information processing apparatus according to (12), wherein
   the cell is a fertilized egg, and
   the recognition feature amount includes at least one of an area of the fertilized egg, a diameter of the fertilized egg, circularity of the fertilized egg, ellipticity of the fertilized egg, a position of center of gravity of the fertilized egg, a central position of the fertilized egg, an area of an inner cell mass of the fertilized egg, a diameter of the inner cell mass of the fertilized egg, circularity of the inner cell mass of the fertilized egg, ellipticity of the inner cell mass of the fertilized egg, a position of center of gravity of the inner cell mass of the fertilized egg, a central position of the inner cell mass of the fertilized egg, an area of a zona pellucida of the fertilized egg, a diameter of the zona pellucida of the fertilized egg, circularity of the zona pellucida of the fertilized egg, ellipticity of the zona pellucida of the fertilized egg, a position of center of gravity of the zona pellucida of the fertilized egg, a central position of the zona pellucida of the fertilized egg, a number of pronuclei of the fertilized egg, an area of the pronuclei of the fertilized egg, a number of polar bodies of the fertilized egg, an area of the polar bodies of the fertilized egg, a number of fragmentations of the fertilized egg, or an area of the fragmentations of the fertilized egg.
(14) The information processing apparatus according to (12), wherein
   the cell is a fertilized egg, and
   the motion feature amount includes at least one of a motion amount of the whole fertilized egg, a motion direction of the whole fertilized egg, a motion amount of blastomeres inside the fertilized egg, a motion amount of an inner cell mass within cells of a blastocyst inside the fertilized egg, or a motion direction of the inner cell mass within cells of the blastocyst inside the fertilized egg.
(15) The information processing apparatus according to (12), wherein
   the cell is a fertilized egg, and
   the morphological feature amount includes at least one of a growth state representing a state of growth of the fertilized egg or a quality condition representing quality of the fertilized egg.
(16) The information processing apparatus according to (12), wherein
   the cell is a fertilized egg, and
   the numerical feature amount includes at least one of an amount of contraction of an area of the fertilized egg, a ratio of contraction of the area of the fertilized egg, contraction speed of the area of the fertilized egg, a contraction period of the area of the fertilized egg, a cumulative amount of movement of the fertilized egg, a number of times of contraction of a total area of the fertilized egg, an amount of contraction of an area of inner cells of the fertilized egg, a ratio of contraction of the area of the inner cells of the fertilized egg, contraction speed of the area of the inner cells of the fertilized egg, a contraction period of the area of the inner cells of the fertilized egg, a number of times of contraction of the area of the inner cells of the fertilized egg, a cleavage period, abnormal cleavage or a compaction period.
(17) The information processing apparatus according to any one of (1) to (16), wherein the control unit further controls display of information indicating an implementation status of the analysis.
(18) An information processing method to be performed by a processor provided at a calculator, the information processing method comprising:
   analyzing a plurality of images obtained by capturing images of an observation target including a cell having ability to divide in chronological order, to calculate a feature amount representing a state of the cell; and
   dynamically controlling an implementation condition of the analysis on a basis of the calculated feature amount.
(19) A program for causing a computer to execute:
   analyzing a plurality of images obtained by capturing images of an observation target including a cell having ability to divide in chronological order, to calculate a feature amount representing a state of the cell; and
   dynamically controlling an implementation condition of the analysis on a basis of the calculated feature amount.
(20) An information processing system comprising:
   an imaging apparatus including at least an imaging unit configured to generate a plurality of images regarding an observation target including a cell having ability to divide by capturing images of the observation target in chronological order; and
   an information processing apparatus including an analyzing unit configured to analyze the plurality of captured images to calculate a feature amount representing a state of the cell, and a control unit configured to dynamically control an implementation condition of the analysis on a basis of the calculated feature amount.

### Reference Signs List

- 1: Information processing system
- 10: Imaging apparatus
- 20: Information processing apparatus
- 30: Display apparatus
- 200: Analyzing unit
- 202: Recognition analyzing unit
- 203: Motion analyzing unit
- 204: Morphological analyzing unit
- 205: Numerical analyzing unit
- 210: Determining unit
- 220: Control unit
- 221: Analysis control unit
- 222: Display control unit
- 230: Storage unit

## Claims

1. An information processing apparatus comprising:
an analyzing unit configured to analyze a plurality of images obtained by capturing images of an observation target including a cell having ability to divide in chronological order, to calculate a feature amount representing a state of the cell; and
a control unit configured to dynamically control an implementation condition of the analysis on a basis of the calculated feature amount.

2. The information processing apparatus according to claim 1, further comprising: a determining unit configured to determine the state of the cell on a basis of the feature amount.

3. The information processing apparatus according to claim 2, wherein the determining unit determines the state of the cell using a classifier constructed in advance.

4. The information processing apparatus according to claim 2, wherein the control unit dynamically controls the implementation condition of the analysis on a basis of the state of the cell determined from the feature amount.

5. The information processing apparatus according to claim 1, wherein the control unit dynamically controls the implementation condition of the analysis on a basis of temporal change of the feature amount.

6. The information processing apparatus according to claim 1, wherein the control unit performs control to interrupt the analysis.

7. The information processing apparatus according to claim 6, wherein the control unit performs control so that a period during which the analysis is not implemented becomes fixed regardless of elapsed time.

8. The information processing apparatus according to claim 6, wherein the control unit performs control so that a period during which the analysis is not implemented becomes longer as time elapses.

9. The information processing apparatus according to claim 1, wherein the control unit performs control to stop implementation of the analysis.

10. The information processing apparatus according to claim 1, wherein the control unit performs control to reduce a number of the feature amounts to be calculated as time elapses.

11. The information processing apparatus according to claim 1, wherein the control unit performs control to reduce a number of predetermined analysis methods to be used in the analysis as time elapses.

12. The information processing apparatus according to claim 1, wherein the feature amount representing the state of the cell includes at least one of a recognition feature amount which is information regarding a shape of the cell, a motion feature amount which is information characterizing motion of the cell, a morphological feature amount which is information characterizing morphology of the cell, or a numerical feature amount which is a numerical value representing a state of a fertilized egg.

13. The information processing apparatus according to claim 12, wherein
the cell is a fertilized egg, and
the recognition feature amount includes at least one of an area of the fertilized egg, a diameter of the fertilized egg, circularity of the fertilized egg, ellipticity of the fertilized egg, a position of center of gravity of the fertilized egg, a central position of the fertilized egg, an area of an inner cell mass of the fertilized egg, a diameter of the inner cell mass of the fertilized egg, circularity of the inner cell mass of the fertilized egg, ellipticity of the inner cell mass of the fertilized egg, a position of center of gravity of the inner cell mass of the fertilized egg, a central position of the inner cell mass of the fertilized egg, an area of a zona pellucida of the fertilized egg, a diameter of the zona pellucida of the fertilized egg, circularity of the zona pellucida of the fertilized egg, ellipticity of the zona pellucida of the fertilized egg, a position of center of gravity of the zona pellucida of the fertilized egg, a central position of the zona pellucida of the fertilized egg, a number of pronuclei of the fertilized egg, an area of the pronuclei of the fertilized egg, a number of polar bodies of the fertilized egg, an area of the polar bodies of the fertilized egg, a number of fragmentations of the fertilized egg, or an area of the fragmentations of the fertilized egg.

14. The information processing apparatus according to claim 12, wherein
the cell is a fertilized egg, and
the motion feature amount includes at least one of a motion amount of the whole fertilized egg, a motion direction of the whole fertilized egg, a motion amount of blastomeres inside the fertilized egg, a motion amount of an inner cell mass within cells of a blastocyst inside the fertilized egg, or a motion direction of the inner cell mass within cells of the blastocyst inside the fertilized egg.

15. The information processing apparatus according to claim 12, wherein
the cell is a fertilized egg, and
the morphological feature amount includes at least one of a growth state representing a state of growth of the fertilized egg or a quality condition representing quality of the fertilized egg.

16. The information processing apparatus according to claim 12, wherein
the cell is a fertilized egg, and
the numerical feature amount includes at least one of an amount of contraction of an area of the fertilized egg, a ratio of contraction of the area of the fertilized egg, contraction speed of the area of the fertilized egg, a contraction period of the area of the fertilized egg, a cumulative amount of movement of the fertilized egg, a number of times of contraction of a total area of the fertilized egg, an amount of contraction of an area of inner cells of the fertilized egg, a ratio of contraction of the area of the inner cells of the fertilized egg, contraction speed of the area of the inner cells of the fertilized egg, a contraction period of the area of the inner cells of the fertilized egg, a number of times of contraction of the area of the inner cells of the fertilized egg, a cleavage period, abnormal cleavage or a compaction period.

17. The information processing apparatus according to claim 1, wherein the control unit further controls display of information indicating an implementation status of the analysis.

18. An information processing method to be performed by a processor provided at a calculator, the information processing method comprising:
analyzing a plurality of images obtained by capturing images of an observation target including a cell having ability to divide in chronological order, to calculate a feature amount representing a state of the cell; and
dynamically controlling an implementation condition of the analysis on a basis of the calculated feature amount.

19. A program for causing a computer to execute:
analyzing a plurality of images obtained by capturing images of an observation target including a cell having ability to divide in chronological order, to calculate a feature amount representing a state of the cell; and
dynamically controlling an implementation condition of the analysis on a basis of the calculated feature amount.

20. An information processing system comprising:
an imaging apparatus including at least an imaging unit configured to generate a plurality of images regarding an observation target including a cell having ability to divide by capturing images of the observation target in chronological order; and
an information processing apparatus including an analyzing unit configured to analyze the plurality of captured images to calculate a feature amount representing a state of the cell, and a control unit configured to dynamically control an implementation condition of the analysis on a basis of the calculated feature amount.
